# EUROPEAN PATENT SPECIFICATION

(11) **EP 4 444 253 B1**
(45) Date of publication and mention of the grant of the patent: **08.10.2025**
(21) Application number: 22839140.5
(22) Date of filing: 07.12.2022
(51) Int. Cl.: A61K 8/06, A61K 8/37, A61K 8/39, A61Q 19/08

(54) **COMPOSITION COMPRISING A COMBINATION OF POLYGLYCEROL ESTERS AND A FILLER**
ZUSAMMENSETZUNG MIT EINER KOMBINATION AUS POLYGLYCEROLESTERN UND EINEM FÜLLSTOFF
COMPOSITION COMPRENANT UNE COMBINAISON D'ESTERS DE POLYGLYCÉROL ET D'UNE CHARGE

(30) Priority: 09.12.2021 FR 2113208
(43) Date of publication of application: 16.10.2024
(73) Proprietor: L'OREAL, 75008 Paris (FR)
(72) Inventor: GUILBAUD, Sophie, 26400 GIGORS ET LOZERON (FR); PIOUD, Noémie, 26400 GIGORS ET LOZERON (FR)
(74) Representative: Ipsilon
(86) International application number: PCT/EP2022/084866
(87) International publication number: WO 2023/104924

(56) References cited:
- EP-B1- 2 814 451
- FR-A1- 2 971 148
- US-B2- 9 132 290

## Description

### Technical field

The present invention relates to the field of caring for and/or making up keratin materials, in particular to the anti-ageing care of keratin materials, notably of the skin.

More precisely, the present invention is directed towards proposing a novel cosmetic composition, in the form of a water-in-oil emulsion, which is particularly advantageous with regard to its technical performance, notably in terms of viscosity, stability and, in particular, stabilization of biological active agents, and the sensory sensations which it provides to the user when said user applies it thereto, in particular to the skin.

For the purposes of the present invention, the term "*keratin materials*" notably denotes the skin, the lips and/or the eyelashes, in particular the skin and/or the lips, and preferably the skin of the body and/or the face, and more preferentially of the face.

### Prior art

Emulsions may be defined as being heterogeneous systems comprising at least two liquid phases that are immiscible or that are very sparingly mutually miscible. In these systems, one of the phases is dispersed in the form of fine droplets in the other phase, so as to observe a mixture that is macroscopically homogeneous to the naked eye.

Conventionally, the formulator of cosmetic compositions uses emulsified systems that combine an aqueous phase for freshness and a fatty phase for comfort.

For example, FR 2 971 148 A1 discloses UV protection compositions comprising high filler contents. They are in the form of water-in-oil (W/O) emulsions and comprise composite metal oxide particles.

US 9 132 290 B2 describes cosmetic W/O emulsions with UV protection, mostly foundations, having good sensory properties and high coverage.

The strong point of these systems is that they enable the combination, within the same composition, of cosmetic ingredients or active agents that have different affinities with respect to these two aqueous and fatty phases, which are immiscible at room temperature.

Emulsifying systems of reverse emulsion type (water-in-oil emulsions, the aqueous phase being in a form dispersed in the continuous fatty phase) have many advantages, with regard to the reduced soapy effect but also the good level of coverage and the homogeneous appearance that they provide compared to direct emulsions (oil-in-water emulsions).

Their weak point is, on the other hand, is that they give a pronounced greasy and tacky feeling, and thus a lack of lightness for the textures obtained.

In order to overcome these drawbacks, it has already been proposed to formulate reverse emulsions comprising a particularly high aqueous phase content, in particular of at least 50.0% by weight, relative to the total weight of the composition. These emulsions are commonly referred to as HIPE (High Internal Phase Emulsion) reverse emulsions.

HIPE reverse emulsions have most particularly advantageous rheological properties, in particular in terms of viscosity, enabling them to be packaged in a jar.

However, the high aqueous phase content of these compositions leads to a high proportion of droplets, which are also larger and therefore closer to each other than in the context of a "conventional" reverse emulsion, comprising an aqueous phase content strictly less than 50.0% by weight.

There is thus, for these compositions, an increased risk of coalescence between the droplets, leading to destabilization of the emulsion.

Combinations of surfactants have already been proposed in the prior art for stabilizing water-in-oil emulsions of HIPE type, for example in WO 2013/120823 and WO 2013/120829.

However, these compositions do not prove entirely satisfactory, in particular with regard to the sensory effect provided.

Moreover, it is known practice to introduce active agents into cosmetic compositions for the purpose of combating the signs of ageing.

Specifically, skin ageing results from the effects of intrinsic and extrinsic factors on the skin. During the ageing process, a detrimental change in the structure and functions of the skin appears. The main clinical signs due to these changes in the skin metabolism are the appearance of wrinkles and fine lines, the cause of which is a slackening and loss of the elasticity of the tissues.

Unfortunately, anti-ageing active agents are often unstable in oxidizing medium and thus very sensitive to certain environmental parameters, for instance light, oxygen and water. Rapid degradation, on the one hand, of these active agents thus follows when they are in contact notably with one of these parameters, which runs counter to the desired efficacy, and, on the other hand, a destabilizing effect on the whole formulation comprising them.

Surprisingly, certain fillers have shown a stabilizing effect on the activated formulation. However, it remains very difficult to use them in large amounts, notably greater than 6% by weight, while at the same time conserving satisfactory sensory results.

Finally, the formulation of environmentally friendly cosmetic products has become a major challenge for meeting new consumer expectations, in particular regarding natural and/or eco-friendly products, i.e. products whose design and development take into account their environmental impacts.

It is thus common practice to seek to replace synthetic or environmentally unfriendly compounds present in cosmetic compositions with natural ingredients and/or ingredients of natural origin.

### Disclosure of the invention

The need thus remains for cosmetic compositions, formulated in the form of water-in-oil emulsions of HIPE type, which combine particularly advantageous technical performance in terms of sensory and rheological properties, while at the same time being stable.

In particular, the need remains for stable water-in-oil emulsions of the HIPE type which provide a good level of sensory pleasure, notably which provide a soft, non-greasy finish on application, which do not lather, and with a substantial reduction in linting and/or the shiny effect on the skin.

The need also remains to formulate such compositions comprising at least one cosmetic active agent, in particular at least one anti-ageing biological active agent, notably in a content of at least 2.0% by weight, without altering their advantageous technical performance, in particular in terms of stability.

Finally, the need also remains to have compositions that are compatible with the current consumer demands, notably regarding the environment.

The present invention specifically aims to meet all or some of these needs.

### Summary of the invention

Thus, according to a first of its aspects, the present invention relates to a composition, notably a cosmetic composition, in particular for making up and/or caring for keratin materials, comprising:
- at least one continuous fatty phase;
- at least one aqueous phase at a concentration of at least 50.0% by weight, relative to the total weight of the composition, and dispersed in said fatty phase;
- at least two esters that are different from each other, chosen from esters of polyglycerol with at least one fatty acid containing from 8 to 30 carbon atoms;
- glyceryl trihydroxystearate; and
- at least 6.0% by weight, relative to the total weight of the composition, of at least one filler chosen from talc, hydrophobic silica aerogel particles, C₈-C₂₂ N-acylamino acid particles, modified or unmodified starches, boron nitride, polymeric fillers, metal oxides and mixtures thereof.

For the purposes of the present invention, the term "*esters that are different from each other*" is intended to denote esters whose chemical composition is at least partially different.

It is understood that the content of filler(s) is expressed as the active material of the components.

For the purposes of the present invention, the term "*fillers*" is intended to denote solid particles of any form, which are in an insoluble and dispersed form in the medium of the composition.

It has already been proposed to stabilize a cosmetic composition in the form of a water-in-oil emulsion of the HIPE type with a combination of specific polyglycerol esters, in a specific mass ratio, and glyceryl trihydroxystearate.

However, the inventors have found, entirely surprisingly, that the presence of specific fillers as defined above in a content of at least 6% by weight in such a cosmetic composition, in the form of a water-in-oil emulsion of HIPE type, in particular moreover comprising at least one cosmetic active agent, such as an anti-ageing biological active agent, advantageously makes it possible to reconcile both excellent stability and excellent sensory performance.

In particular, the cosmetic compositions thus obtained have good rheological properties, notably in terms of viscosity and/or texture, and particularly advantageous cosmetic properties in terms of reduced greasiness, shine and tack, or improved freshness and moisturizing effects, during application and after application.

These effects are all the more surprising since it is generally accepted that the use of fillers in cosmetic compositions, such as diffusing fillers or fillers with a soft-focus effect, in particular in large amounts, can lead to destabilization of said compositions and induce cosmetic properties that are at variance with the users' expectations.

In addition, these effects are obtained by reducing the content of, or even by dispensing with, starting materials which are harmful to the environment or which generate substances that are harmful to the environment in the composition.

For the purposes of the invention, the term "*stable emulsion*" means an emulsion which, after 2 months of storage at 4°C, at room temperature (i.e. between 20 and 25°C), at 37°C and at 45°C, in particular at 45°C, does not show any macroscopic change in colour, odour or viscosity, but which, on the contrary, remains homogeneous and uniform, and which does not undergo phase separation, (no separation of the aqueous phase from the oily phase) or release any oil.

The stability of the composition can also be assessed by examining the defects of the composition after several cycles of temperature change over 10 days and also by a vibration test to simulate transport stability.

Advantageously, a composition according to the invention does not comprise any controversial ingredients, and in particular does not comprise any silicone compounds, such as silicone oils.

A composition according to the invention is used in particular for caring for and/or making up keratin materials, and preferably for caring for keratin materials.

Thus, according to another of its aspects, the invention further relates to a cosmetic process for making up and/or caring for, preferably for caring for, keratin materials, in particular the skin, comprising at least one step of applying a composition according to the invention to said keratin materials.

It is understood that the processes and methods defined according to the present invention are non-therapeutic.

Other characteristics, variants and advantages of the compositions according to the invention will emerge more clearly on reading the description and the examples that follow.

### Detailed description

### Composition

As stated previously, a composition according to the invention may advantageously be cosmetic.

A composition according to the invention is generally suitable for topical application to the skin and thus generally comprises a physiologically acceptable medium, i.e. a medium that is compatible with the skin.

It is preferably a cosmetically acceptable medium, i.e. a medium which has a pleasant colour, odour and feel and which does not cause any unacceptable discomfort, i.e. stinging, tautness or redness, liable to discourage the user from applying this composition.

A cosmetic composition according to the invention is in the form of a reverse emulsion of HIPE type, namely comprising a continuous fatty phase and an aqueous phase in a content of at least 50% by weight, dispersed in said fatty phase.

In particular, a composition according to the invention preferably has a viscosity ranging from 0.5 to 12 Pa.s, preferably from 1.0 to 10 Pa.s, more preferentially from 2.5 to 8.0 Pa.s, for example from 5.0 to 8.0 Pa.s.

The viscosity of the composition is measured at 25°C using a Rheomat RM100 Touch^{®} (from the company LAMY) equipped with an MS-R4 spindle rotating at a rotational speed of 200 rpm. The measurement is taken after 10 minutes of rotation. The viscosity measurements are taken at most 1 week after production.

A composition according to the invention can be packaged in a jar.

A composition according to the invention may be prepared according to the techniques that are well known to those skilled in the art.

### Esters of polyglycerol with at least one fatty acid

As mentioned above, a composition according to the invention comprises at least two esters that are different from each other, chosen from esters of polyglycerol with at least one fatty acid containing from 8 to 30 carbon atoms.

For the purposes of the present invention, the term "ester of polyglycerol with at least one fatty acid containing from 8 to 30 carbon atoms" means an ester obtained from at least one fatty acid and polyglycerol, in which the fatty acid comprises a linear or branched, saturated or unsaturated, preferably aliphatic, hydrocarbon-based group comprising from 8 to 30 carbon atoms, preferably from 16 to 20 carbon atoms.

Advantageously, said fatty acids comprising from 8 to 30 carbon atoms are chosen from stearic acid, isostearic acid, lauric acid, myristic acid, palmitic acid, palmitoleic acid, arachidic acid, arachidonic acid, behenic acid, lignoceric acid, cerotic acid, and mixtures thereof.

Preferably, the fatty acids used in the present invention comprise a hydrocarbon-based chain, which is preferably branched, comprising from 16 to 20 carbon atoms, and are independently chosen from stearic acid, isostearic acid, palmitic acid and arachidic acid.

According to one embodiment, at least one of the fatty acids is in a polymeric form, as is the case, for example, for polyhydroxystearic acid (polymer of 12-hydroxystearic acid).

Advantageously, the fatty acids are identical (the glycerol part then being different) or the fatty acids are different (the glycerol part possibly being identical or different), and preferably chosen independently from stearic acid, isostearic acid, poly(12-hydroxystearic acid) and mixtures thereof.

The term "polyglycerol" means a compound having the following formula: 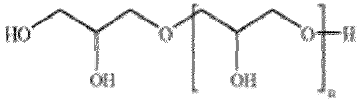 in which the degree of condensation n ranges from 1 to 11, preferably from 2 to 6 and even more preferentially from 3 to 6.

Preferably, at least one of the polyglycerol fatty acid esters contains 2 to 15 glycerol units, preferably from 2 to 10 glycerol units, in particular from 3 to 5 glycerol units.

According to one embodiment, the polyglycerol esters present in a composition according to the invention have an HLB, measured at 25°C, of less than or equal to 8.0, preferably between 0.1 and 8.0, more preferentially between 2.0 and 8.0.

The HLB (hydrophilic-lipophilic balance) is the ratio between the hydrophilic part and the lipophilic part in the surfactant molecule. This term is well known to those skilled in the art and is described, for example, in "The HLB system, A time-saving guide to Emulsifier Selection", published by ICI Americas Inc., 1984. The HLB of the surfactant(s) used according to the invention may be determined via the Griffin method or the Davies method. The esters of polyglycerol with at least one fatty acid containing from 8 to 30 carbon atoms are advantageously present in a composition according to the invention in a content ranging from 0.5% to 15% by weight, preferably from 1.5% to 10% by weight, more preferentially from 2.5% to 8.0% by weight, relative to the total weight of the composition.

According to a particular embodiment, a composition according to the invention comprises, as esters of polyglycerol with at least one fatty acid containing from 8 to 30 carbon atoms:
- at least one ester of polyglycerol with at least one poly(hydroxystearic acid) and carboxylic acids, resulting from the esterification between at least one polyglycerol and (i) at least one poly(hydroxystearic acid); (ii) at least one di- and/or tricarboxylic acid; and (iii) at least one saturated or unsaturated, linear or branched fatty acid containing from 6 to 22 carbon atoms, known as "esters of polyglycerol with a poly(hydroxystearic acid)"; and
- at least one ester of polyglycerol including from 4 to 15 glycerol units and of at least one saturated or unsaturated, linear or branched fatty acid including from 6 to 24 carbon atoms, known as "esters of polyglycerol and of a fatty acid".

### Polyglycerol esters with a poly(hydroxystearic acid)

As ester of polyglycerol with a poly(hydroxystearic acid) and carboxylic acids, mention may be made more particularly of esters derived from the reaction of a mixture of polyglycerol with (i) at least one poly(hydroxystearic acid) with from 1 to 10, preferably from 2 to 8, even more preferentially from 2 to 5 polyglycerol units (preferably 4 units); (ii) at least one linear or branched aliphatic dicarboxylic acid containing from 2 to 16 carbon atoms, preferably from 4 to 14 carbon atoms (preferably sebacic acid); and (iii) at least one saturated or unsaturated, linear or branched fatty acid containing from 6 to 22 carbon atoms, preferably from 16 to 20 carbon atoms (preferably isostearic acid). Advantageously, the degree of esterification of the polyglycerol mixture is between 20% and 40%, preferably between 40% and 70%.

According to one preferred embodiment, the polyglycerol ester of a poly(hydroxystearic acid) is derived from the esterification between a mixture of polyglycerol and (i) a poly(hydroxystearic acid) with 4 polyglycerol units; (ii) linear or branched aliphatic dicarboxylic acids containing 8 to 12 carbon atoms; and (iii) saturated or unsaturated, linear or branched fatty acids containing from 16 to 20 carbon atoms.

As a preferred example of a poly(hydroxystearic acid) ester of polyglycerol, mention may be made of polyglyceryl-4 diisostearate/polyhydroxystearate/sebacate of formula: 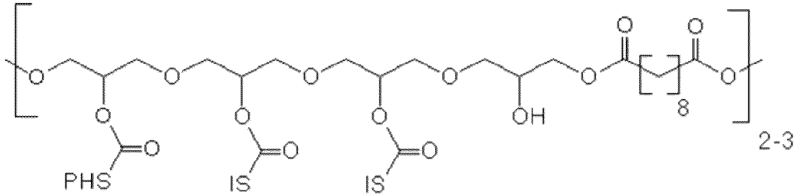 in which PHS denotes polyhydroxystearic acid and IS denotes isostearic acid.

Such a polyglyceryl-4 diisostearate/polyhydroxystearate/sebacate compound is sold, for example, under the name Isolan^{®} GPS by the company Evonik Goldschmidt.

The polyglycerol ester of a poly(hydroxystearic acid) and carboxylic acids according to the invention may advantageously be used as a mixture with other esters of polyglycerol, in particular comprising 3 polyglycerol units, with at least one fatty acid, in particular an oleic acid.

Even more preferably, use will be made in the composition of the invention of the Polyglyceryl-4 Diisostearate/Polyhydroxystearate/Sebacate (and) Caprylic/Capric Triglyceride (and) Polyglyceryl-3 Oleate (and) Diisostearoyl Polyglyceryl-3 Dimer Dilinoleate mixture sold under the name Isolan^{®} 17 MB by the company Evonik Goldschmidt.

A composition according to the invention advantageously comprises between 0.5% and 5.0% by weight, preferably between 1.0% and 4.0% by weight, more preferentially between 1.5% and 3.5% by weight, of at least one polyglycerol ester of a poly(hydroxystearic acid) and carboxylic acids as defined above, relative to the total weight of the composition.

### Polyglycerol fatty acid esters

Preferably, the ester(s) of polyglycerol and a fatty acid are chosen from esters resulting from the reaction of polyglycerol comprising from 4 to 12 glycerol units, preferably from 4 to 10 glycerol units and a saturated or unsaturated, linear or branched fatty acid containing from 8 to 22 carbon atoms, preferably from 8 to 20 carbon atoms, more preferably from 12 to 20 carbon atoms, even more preferably from 16 to 20 carbon atoms.

As examples of fatty acids that are suitable for the synthesis of the polyglycerol ester of at least one fatty acid according to the invention, mention may be made of isostearic acid, stearic acid, linoleic acid, oleic acid, behenic acid, myristic acid, lauric acid, capric acid and mixtures thereof.

Preferably, the polyglycerol fatty acid ester(s) are chosen from the esters derived from the esterification reaction between a polyglycerol comprising from 4 to 5 glycerol units, in particular comprising 4 glycerol units, and at least one aliphatic monocarboxylic acid comprising an alkyl chain containing from 16 to 20 carbon atoms, such as a stearyl and/or isostearyl chain.

Mention may be made, for example, of the ester derived from the reaction of polyglycerol-4 (glycerol homopolymer comprising 4 glycerol units) and isostearic acid (INCI name: polyglyceryl-4 isostearate) such as the product sold under the name Isolan^{®} GI34 by the company Evonik Goldschmidt.

A composition according to the invention advantageously comprises between 0.1% and 5.0% by weight, preferably between 0.5% and 4.0% by weight, more preferentially between 1.0% and 4.0% by weight, of at least one polyglycerol fatty acid ester, relative to the total weight of the composition.

Advantageously, the mass ratio of ester of polyglycerol with at least one poly(hydroxystearic acid) and carboxylic acids/ester of a polyglycerol and at of least one fatty acid is strictly greater than 0.7. It is preferably greater than or equal to 0.8, more preferentially greater than or equal to 1. In particular, the mass ratio of ester of polyglycerol with at least one poly(hydroxystearic acid) and carboxylic acids/ester of a polyglycerol and at of least one fatty acid may be between 0.8 and 2.0.

It is understood that the mass ratio is expressed as active material of the components.

### Glyceryl trihydroxystearate

As mentioned above, a composition according to the invention comprises glyceryl trihydroxystearate (INCI name: trihydroxystearin).

Glyceryl trihydroxystearate is a compound of formula (I):

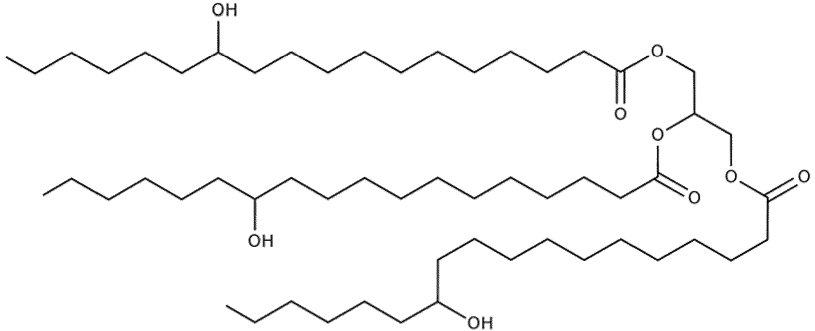

It is a wax, namely a lipophilic compound, which is solid at room temperature (25°C), with a reversible solid/liquid change of state, which has a melting point of greater than or equal to 30°C, in particular greater than 60°C.

For the purposes of the invention, the melting point corresponds to the temperature of the most endothermic peak observed on thermal analysis (DSC) as described in the standard ISO 11357-3; 1999. The melting point of the wax may be measured using a differential scanning calorimeter (DSC), for example the calorimeter sold under the name MDSC 2920 by the company TA Instruments.

A composition according to the invention advantageously comprises between 0.01% and 2.0% by weight, in particular between 0.1% and 1.5% by weight, preferably between 0.1% and 1.0% by weight, more preferentially between 0.2% and 0.8% by weight, of glyceryl trihydroxystearate, relative to the total weight of the composition.

### Fillers

As mentioned previously, a composition according to the invention comprises at least 6.0% by weight of at least one filler chosen from talc, hydrophobic silica aerogel particles, C₈-C₂₂ N-acylamino acid particles, modified or unmodified starches, boron nitride, polymeric fillers, metal oxides and mixtures thereof.

A filler content of at least 6% by weight, in particular of at least 10% by weight, relative to the total weight of the composition, advantageously makes it possible to formulate a composition according to the invention having both satisfactory stability and satisfactory sensory properties.

Moreover, the inventors have found that the nature of the filler has an influence on the rheological properties and/or the sensory properties of the composition. More precisely, the fillers required according to the invention advantageously make it possible to formulate a water-in-oil emulsion of HIPE type having a good compromise between fluidity and thickness, not leaving a greasy and/or shiny finish after application.

### Talc

As an example of talcs that are suitable as filler according to the invention, mention may be made of those sold under the names Luzenac Pharma Umo (mean size 2.7 pm) by the company World Minerals (Imerys).

### Hydrophobic silica aerogel particles

Silica aerogels are porous materials obtained by replacing (by drying) the liquid component of a silica gel with air.

They are generally synthesized by means of a sol-gel process in a liquid medium and then dried, usually by extraction with a supercritical fluid, the one most commonly used being supercritical CO₂. This type of drying makes it possible to avoid shrinkage of the pores and of the material.

The hydrophobic silica aerogel particles used in the present invention preferably have a specific surface area per unit mass (SM) ranging from 500 to 1500 m²/g, preferably from 600 to 1200 m²/g and better still from 600 to 800 m²/g, and a size expressed as the volume-mean diameter (D[0.5]) ranging from 1 to 1500 µm, better still from 1 to 1000 µm, preferably from 1 to 100 µm, in particular from 1 to 30 µm, more preferably from 5 to 25 µm, better still from 5 to 20 µm and even better still from 5 to 15 µm.

The specific surface area per unit mass may be determined by the nitrogen absorption method, known as the BET (Brunauer-Emmett-Teller) method, corresponding to international standard ISO 5794/1 (annex D).

The sizes of the silica aerogel particles may be measured by static light scattering using a commercial particle size analyser such as the MasterSizer 2000 machine from Malvern. The data are processed on the basis of the Mie scattering theory.

The aerogels used according to the present invention are aerogels of hydrophobic silica, preferably of silylated silica (INCI name: silica silylate).

The term "*hydrophobic silica*" means any silica whose surface is treated with silylating agents, for example with halogenated silanes such as alkylchlorosilanes, siloxanes, in particular dimethylsiloxanes such as hexamethyldisiloxane, or silazanes, so as to functionalize the OH groups with silyl groups Si-Rn, for example trimethylsilyl groups.

As regards the preparation of hydrophobic silica aerogel particles surface-modified by silylation, reference may be made to US 7 470 725.

Use will preferably be made of hydrophobic silica aerogel particles surface-modified with trimethylsilyl groups, preferably having the INCI name Silica silylate.

As hydrophobic silica aerogels that may be used in the invention, an example that may be mentioned is the aerogel sold under the name VM-2260^{®} or VM-2270^{®} (INCI name: Silica silylate) by the company Dow Corning, the particles of which have a mean size of about 1000 microns and a specific surface area per unit mass ranging from 600 to 800 m²/g. Mention may also be made of the aerogels sold by the company Cabot under the references Aerogel TLD 201, Aerogel OGD 201 and Aerogel TLD 203, Enova^{®} Aerogel MT 1100 and Enova^{®} Aerogel MT 1200.

The silica aerogel particles, in particular of silylated silica aerogel, may be present in a composition according to the invention in a content ranging from 0.5% to 13%, preferably in a content ranging from 1% to 5% by weight and in particular ranging from 1% to 2% by weight relative to the total weight of the composition.

### C₈-C₂₂ N-acylamino acid particles

N-Acylamino acids that are suitable as fillers according to the invention comprise an acyl group containing from 8 to 22 carbon atoms, in particular a 2-ethyl hexanoyl, caproyl, lauroyl, myristoyl, palmitoyl, stearoyl or cocoyl group, preferably lauroyl.

The amino acid may be, for example, lysine, glutamic acid or alanine, preferably lysine. According to a preferred embodiment, the C₈-C₂₂ N-acylamino acid particles are lauroyllysine particles.

By way of example, mention may be made of the lauroyllysine powder sold under the name Amihope LL^{®} (mean size 11.7 pm) by the company Ajinomoto.

The C₈-C₂₂ N-acylamino acid particles may be present in a composition according to the invention in a content ranging from 5% to 50% by weight, relative to the total weight of the composition, preferably from 5% to 40% by weight, more preferentially from 6% to 25% by weight.

### Starches

The starch molecules that are suitable as fillers according to the present invention may originate from all plant sources of starch, notably cereals, vegetables and tubers. More particularly, they may be corn, rice, cassava, barley, potato, wheat, sorghum, pea, oat or tapioca starches. The starch is preferably derived from corn.

Examples that may be mentioned notably include the Remy DR I^{®} rice starch sold by the Remy company; B^{®} Corn Starch from the company Roquette; potato starch modified with 2-chloroethyl aminodipropionic acid neutralized with sodium hydroxide, sold under the name Structure Solanace^{®} by the company National Starch; the native tapioca starch powder sold under the name Tapioca Pure^{®} by the company National Starch.

The modified starch may be a (C₁-C₄) carboxyalkyl starch, also referred to as "carboxyalkylstarch". These compounds are obtained by grafting carboxyalkyl groups onto one or more alcohol functions of starch, notably by reaction of starch and of an alkali metal (such as sodium) monochloroacetate in alkaline (basic) medium.

The carboxyalkyl starches are advantageously used in the form of salts and notably of salts of alkali metals or alkaline-earth metals such as Na, K, Li, NH₄, or salts of a quaternary ammonium or of an organic amine such as monoethanolamine, diethanolamine or triethanolamine. The C₁-C₄ carboxyalkyl starches are preferably carboxymethyl starches.

The carboxyalkyl starches that may be used according to the present invention are preferably sodium salts of carboxymethyl starch (INCI name: Sodium Carboxymethyl Starch), in particular a sodium salt of carboxymethyl starch, such as those sold under the name Primojel^{®} by the company DMV International or Glycolys^{®} and Glycolys^{®} LV by the company Roquette.

The modified or unmodified starch(es) may be present in a composition according to the invention in a content ranging from 1% to 8% by weight and preferably ranging from 2% to 6% by weight relative to the total weight of the composition.

### Boron nitride

Boron nitride is a chemical compound having the formula BN.

Boron nitride preferably has a number-average primary size of between 1 and 50 µm, better still between 1 and 30 µm, more preferentially between 1 and 25 µm, and even more preferentially between 5 and 15 µm.

For the purposes of the present invention, the term "primary particle size" means the maximum dimension that it is possible to measure between two diametrically opposite points of an individual particle. The size may be determined, for example, by transmission electron microscopy or by measuring the specific surface area via the BET method or else with a laser particle size analyser.

As commercial boron nitrides that may be used in a composition according to the invention, mention may notably be made of the boron nitrides sold by Saint Gobain Ceramics, notably under the names PUHP3002, PUHP3008 or PUHP1030L, or else Softouch BN CC6058 Powder from Momentive Performance Materials.

Boron nitride may be present in a composition according to the invention in a content ranging from 1.0% to 8.0% by weight, and preferably from 2.0% to 6.0% by weight, relative to the total weight of the composition.

### Polymeric fillers

For the purposes of the present invention, the term "polymeric filler" is intended to denote any filler formed from crosslinked or non-crosslinked particles of polymer(s), including homopolymers and copolymers.

Such polymeric fillers are, for example, poly-β-alanine and polyethylene powders, tetrafluoroethylene polymer (Teflon^{®}) powders, hollow polymeric microspheres, in particular polyvinylidene chloride/acrylonitrile such as Expancel^{®} (Nobel Industrie), (co)polymers of acrylic acid such as the hollow spherical polymethyl methacrylate (PMMA) powders sold under the names Covabead LH85 (mean size 7.5 pm) by the company Sensient or Techpolymer MBP-8 (mean size 7.5 pm) by the company Sekisui Plastics, silicone resin microbeads (for example Tospearls^{®} by Toshiba), polyurethane powders, in particular, crosslinked polyurethane powders comprising a copolymer, said copolymer comprising trimethylol hexyl lactone, elastomeric polyorganosiloxane particles optionally coated with silicone resin, notably silsesquioxane resin, such as the products sold under the name KSP-100^{®} (mean size 4.3 pm) or KSP-300^{®} (mean size 6 pm), by the company Shin-Etsu, (INCI name: Vinyl 15 Dimethicone/Methicone Silsesquioxane Crosspolymer), organosilicon particle powders, for example in the form of bowls or rugby balls such as those described in JP-2003 128 788, JP-A-2000- 191789, or in patent application EP1579841 and sold notably under the names NLK506^{©} (mean size 2.6 pm) and NLK602^{©} (mean size 2.3 pm) by the company Takemoto Oil & Fat, polyamide powders, in particular Nylon 12, those sold under the name Orgasol^{®} 2002 (mean size 9 pm) by the company Arkema, or the ethylene glycol dimethacrylate/lauryl methacrylate copolymer powder sold under the name Polytrap^{®} 6603 (mean size 12.2 pm) by the company Dow Corning.

### Metal oxides

Fillers that are suitable for use in the invention may also be chosen from metal oxides, for example iron oxide, zinc oxide, titanium oxide, zirconium oxide or aluminium oxide, preferably iron oxide.

The metal oxide(s) may be present in a composition according to the invention in a content ranging from 5% to 25% by weight and preferably ranging from 7% to 15% by weight relative to the total weight of the composition.

A composition according to the invention preferably comprises at least 6% by weight, relative to the total weight of the composition, of at least one filler chosen from talc, hydrophobic silica aerogel particles, C₈-C₂₂ N-acylamino acid particles, modified or unmodified starches, boron nitride, and mixtures thereof, more preferentially chosen from C₈-C₂₂ N-acylamino acid particles, modified or unmodified starches, and mixtures thereof.

Advantageously, a composition according to the invention comprises at least 6% by weight, relative to the total weight of the composition, of at least one filler chosen from lauroyl lysine, corn starch, and mixtures thereof, preferably a mixture of lauroyl lysine and corn starch.

Preferably, a composition according to the invention comprises at least 10% by weight of at least one filler as defined above.

In particular, such fillers may be present in a composition according to the invention in a content of between 6.0% and 60% by weight, notably between 8.0% and 50% by weight, in particular between 10% and 30% by weight, relative to the total weight of the composition. According to a particular embodiment, a composition according to the invention comprises at least, as fillers:
- lauroyl lysine, in a content ranging from 5.0% to 50% by weight, preferably from 8.0% to 25% by weight; and
- at least one corn starch, in a content ranging from 1.0% to 8.0% by weight, preferably from 1.5% to 5.0% by weight,
the contents being expressed relative to the total weight of the composition.

### Fatty phase

As mentioned above, a composition according to the invention comprises at least one continuous fatty phase.

The fatty phase preferably contains at least one oil, notably a cosmetic oil. It may also contain other fatty substances.

The term "oil" means a water-immiscible non-aqueous compound that is liquid at room temperature (20°C) and at atmospheric pressure (760 mmHg).

A fatty phase that is suitable for preparing the compositions, notably cosmetic compositions, according to the invention may comprise hydrocarbon-based oils or silicone oils, which may or may not be fluorinated, or mixtures thereof.

Preferably, a composition according to the invention comprises less than 2.0% by weight of silicone oil(s), in particular less than 1.0% by weight of silicone oil(s), preferably less than 0.5% by weight, relative to the total weight of the composition, and more preferentially is free of silicone oil(s).

A composition comprising a limited content of silicone oil(s) is advantageously more natural, but also lighter, less tacky and less rough to the touch, with a softer finish, than a composition comprising 2% by weight or more of silicone oil(s), relative to the total weight of the composition.

The oils may be volatile or non-volatile.

They may be of animal, plant, mineral or synthetic origin.

The term "non-volatile" refers to an oil whose vapour pressure at room temperature and atmospheric pressure is non-zero and is less than 10⁻³ mmHg (0.13 Pa).

For the purposes of the present invention, the term "silicone oil" means an oil comprising at least one silicon atom, and notably at least one Si-O group.

The term "fluoro oil" refers to an oil comprising at least one fluorine atom.

The term "hydrocarbon-based oil" means an oil mainly containing hydrogen and carbon atoms and which may optionally comprise one or more, preferably one, carbonate or ester group.

The oils may optionally comprise oxygen, nitrogen, sulfur and/or phosphorus atoms, for example in the form of hydroxyl or acid radicals.

For the purposes of the invention, the term "volatile oil" refers to any oil that is capable of evaporating on contact with the skin in less than one hour, at room temperature and atmospheric pressure. The volatile oil is a volatile cosmetic compound, which is liquid at room temperature, notably having a non-zero vapour pressure, at room temperature and atmospheric pressure, notably having a vapour pressure ranging from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

Mention may notably be made of volatile hydrocarbon-based oils containing from 8 to 16 carbon atoms, branched C₈-C₁₆ alkanes, for instance C₈-C₁₆ isoalkanes (also known as isoparaffins), isododecane, isodecane, isohexadecane and, for example, the oils sold under the trade names Isopar or Permethyl, branched C₈-C₁₆ esters, for instance isohexyl neopentanoate, and mixtures thereof. In particular, the volatile hydrocarbon-based oil is chosen from volatile hydrocarbon-based oils containing from 8 to 16 carbon atoms and mixtures thereof.

Mention may also be made of volatile linear alkanes comprising from 8 to 16 carbon atoms, in particular from 10 to 15 carbon atoms and more particularly from 11 to 13 carbon atoms, for instance n-dodecane (C₁₂) and n-tetradecane (C₁₄) sold by Sasol under the respective references Parafol^{®} 12-97 and Parafol^{®} 14-97, and also mixtures thereof, the undecane-tridecane mixture, mixtures of n-undecane (C₁₁) and of n-tridecane (C₁₃) obtained in examples 1 and 2 of patent application WO 2008/155 059 from the company Cognis, and mixtures thereof.

The following mixtures of linear or branched alkanes, preferably of plant origin, may also be mentioned:
- a mixture of C₁₅-C₁₉ branched alkanes, for example the product sold by the company SEPPIC under the name Emogreen^{®} L15;
- a mixture of C₁₅-C₁₉ linear and/or branched alkanes, for example the product sold by the company SEPPIC under the name Emogreen^{®} L19.

Preferably, a composition according to the invention may comprise at least one hydrocarbon-based oil chosen from volatile linear alkanes comprising from 11 to 13 carbon atoms, in particular an undecane-tridecane mixture, and linear and/or branched C₁₅-C₁₉ alkanes, in particular a mixture of linear and/or branched C₁₅-C₁₉ alkanes.

In particular, such hydrocarbon-based oils may be present in a composition according to the invention in a content ranging from 2.0% to 20.0% by weight and preferably from 3.0% to 15.0% by weight, relative to the total weight of the composition.

Volatile silicone oils that may be mentioned include volatile linear silicone oils such as hexamethyldisiloxane, octamethyltrisiloxane, decamethyltetrasiloxane, tetradecamethylhexasiloxane, hexadecamethylheptasiloxane and dodecamethylpentasiloxane.

Volatile cyclic silicone oils that may be mentioned include hexamethylcyclotrisiloxane, octamethylcyclotetrasiloxane, decamethylcyclopentasiloxane, cyclohexasiloxane and dodecamethylcyclohexasiloxane, and in particular cyclohexasiloxane.

Mention may also be made of nonvolatile hydrocarbon-based, fluoro and/or silicone oils. Non-volatile hydrocarbon-based oils that may notably be mentioned include:
- hydrocarbon-based oils of animal origin,
- hydrocarbon-based oils of plant origin, such as squalane, and synthetic ethers containing from 10 to 40 carbon atoms, such as dicaprylyl ether,
- synthetic esters, for instance the oils of formula R₁COOR₂, in which R₁ represents a linear or branched fatty acid residue including from 1 to 40 carbon atoms and R₂ represents a hydrocarbon-based chain that is notably branched, containing from 1 to 40 carbon atoms, on condition that R₁ + R₂ is greater than or equal to 10. The esters may notably be chosen from esters of alcohol and of fatty acid, for instance cetostearyl octanoate, esters of isopropyl alcohol, such as isopropyl myristate, isopropyl palmitate, ethyl palmitate, 2-ethylhexyl palmitate, isopropyl stearate, octyl stearate, hydroxylated esters, for instance isostearyl lactate, octyl hydroxystearate, alcohol or polyalcohol ricinoleates, hexyl laurate, neopentanoic acid esters, for instance isodecyl neopentanoate, isotridecyl neopentanoate, isononanoic acid esters, for instance isononyl isononanoate, isotridecyl isononanoate and octyl isononanoate, oleyl erucate, isopropyl lauroyl sarcosinate, diisopropyl sebacate, isocetyl stearate, isodecyl neopentanoate or isostearyl behenate;
- polyol esters and pentaerythritol esters, for instance dipentaerythrityl tetrahydroxystearate/tetraisostearate,
- fatty alcohols that are liquid at room temperature, with a branched and/or unsaturated carbon-based chain containing from 12 to 26 carbon atoms, for instance 2-octyldodecanol, isostearyl alcohol and oleyl alcohol,
- C₁₂-C₂₂ higher fatty acids, such as oleic acid, linoleic acid, linolenic acid, and mixtures thereof,
- carbonates, such as dicaprylyl carbonate,
- non-phenyl silicone oils, for instance caprylyl methicone, and
- phenyl silicone oils, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates, dimethicones or phenyl trimethicone with a viscosity of less than or equal to 100 cSt, trimethylpentaphenyltrisiloxane, and mixtures thereof;
and also mixtures of these various oils.

In particular, the composition may also comprise at least one non-volatile oil, chosen in particular from non-volatile apolar hydrocarbon-based oils.

For the purposes of the present invention, the term "apolar oil" means an oil whose solubility parameter at 25°C, δₐ, is equal to 0 (J/cm³)^{½}.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation δₐ = (δp² + δh²)^{½}.

The parameters δₚ, δₕ, δ_{D} and δₐ are expressed in (J/cm³)^{½}.

The non-volatile apolar hydrocarbon-based oil is free of oxygen atoms.

Preferably, the non-volatile apolar hydrocarbon-based oil may be chosen from linear or branched hydrocarbons of mineral or synthetic origin. In particular, it may be chosen from:
- liquid paraffin or derivatives thereof,
- liquid petroleum jelly,
- polybutylenes, for example Indopol H-100 (molar mass or Mw = 965 g/mol), Indopol H-300 (Mw = 1340 g/mol) and Indopol H-1500 (Mw = 2160 g/mol) sold or manufactured by the company Amoco,
- polyisobutenes and hydrogenated polyisobutenes, for example Parleam^{®} sold by the company Nippon Oil Fats, Panalane H-300 E sold or manufactured by the company Amoco (Mw = 1340 g/mol), Viseal 20000 sold or manufactured by the company Synteal (Mw = 6000 g/mol) and Rewopal PIB 1000 sold or manufactured by the company Witco (Mw = 1000 g/mol),
- decene/butene copolymers, polybutene/polyisobutene copolymers, for example Indopol L-14,
- polydecenes and hydrogenated polydecenes, for example Puresyn 10 (Mw = 723 g/mol) and Puresyn 150 (Mw = 9200 g/mol) sold or manufactured by the company Mobil Chemicals,
- and mixtures thereof.

Said non-volatile oil may also be an ester oil, in particular containing between 18 and 70 carbon atoms.

Examples that may be mentioned include monoesters, diesters or triesters.

The ester oils may notably be hydroxylated.

The non-volatile ester oil may preferably be chosen from:
- monoesters comprising between 18 and 40 carbon atoms in total, in particular the monoesters of formula R₁COOR₂ in which R₁ represents a linear or branched fatty acid residue including from 4 to 40 carbon atoms and R₂ represents a hydrocarbon-based chain that is notably branched, containing from 4 to 40 carbon atoms, on condition that R₁ + R₂ is greater than or equal to 18, for instance Purcellin oil (cetostearyl octanoate), isononyl isononanoate, C₁₂ to C₁₅ alkyl benzoate, 2-ethylhexyl palmitate, octyldodecyl neopentanoate, 2-octyldodecyl stearate, 2-octyldodecyl erucate, isostearyl isostearate, diisopropyl sebacate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate, 2-diethylhexyl succinate or isoamyl laurate. Preferably, they are esters of formula R₁COOR₂ in which R₁ represents a linear or branched fatty acid residue including from 4 to 40 carbon atoms and R₂ represents a hydrocarbon-based chain that is notably branched, containing from 4 to 40 carbon atoms, R₁ and R₂ being such that R₁ + R₂ is greater than or equal to 18. Preferably, the ester comprises between 18 and 40 carbon atoms in total. Preferred monoesters that may be mentioned include isononyl isononanoate, oleyl erucate and/or 2-octyldodecyl neopentanoate;
- diesters, notably comprising between 18 and 60 carbon atoms in total and in particular between 18 and 50 carbon atoms in total. It is notably possible to use diesters of dicarboxylic acids and of monoalcohols, preferably such as diisostearyl malate, or glycol diesters of monocarboxylic acids, such as neopentyl glycol diheptanoate or polyglyceryl-2 diisostearate, notably such as the compound sold under the trade reference Dermol DGDIS by the company Akzo;
- triesters, notably comprising between 35 and 70 carbon atoms in total, in particular such as triesters of a tricarboxylic acid, such as triisostearyl citrate, or tridecyl trimellitate, or glycol triesters of monocarboxylic acids such as polyglyceryl-2 triisostearate;
- tetraesters, notably with a total carbon number ranging from 35 to 70, such as pentaerythritol or polyglycerol tetraesters of a monocarboxylic acid, for instance pentaerythrityl tetrapelargonate, pentaerythrityl tetraisostearate, pentaerythrityl tetraisononanoate, glyceryl tris(2-decyl)tetradecanoate, polyglyceryl-2 tetraisostearate or pentaerythrityl tetrakis(2-decyl)tetradecanoate;
- polyesters obtained by condensation of unsaturated fatty acid dimer and/or trimer and of diol, such as those described in patent application FR 0 853 634, in particular such as of dilinoleic acid and of 1,4-butanediol. Mention may notably be made in this respect of the polymer sold by Biosynthis under the name Viscoplast 14436H (INCI name: dilinoleic acid/butanediol copolymer), or else copolymers of polyols and of dimer diacids, and esters thereof, such as Hailuscent ISDA;
- esters and polyesters of diol dimer and of monocarboxylic or dicarboxylic acid, such as esters of diol dimer and of fatty acid and esters of diol dimer and of dicarboxylic acid dimer, in particular which may be obtained from a dicarboxylic acid dimer derived in particular from the dimerization of an unsaturated fatty acid notably of C₈ to C₃₄, notably of C₁₂ to C₂₂, in particular of C₁₆ to C₂₀ and more particularly of C₁₈, such as esters of dilinoleic diacids and of dilinoleic diol dimers, for instance those sold by the company Nippon Fine Chemical under the trade names Lusplan DD-DA5^{®} and DD-DA7^{®};
- vinylpyrrolidone/1-hexadecene copolymers, for instance the product sold under the name Antaron V-216 (also known as Ganex V216) by the company ISP (Mw = 7300 g/mol);
- hydrocarbon-based plant oils such as fatty acid triglycerides (which are liquid at room temperature), notably of fatty acids containing from 7 to 40 carbon atoms, such as heptanoic or octanoic acid triglycerides or jojoba oil; mention may be made in particular of saturated triglycerides such as caprylic/capric triglyceride, glyceryl triheptanoate, glyceryl trioctanoate, and C₁₈-₃₆ acid triglycerides such as those sold under the reference DUB TGI 24 by Stéarinerie Dubois, and unsaturated triglycerides such as castor oil, olive oil, ximenia oil and pracaxi oil;
- and mixtures thereof.

Preferably, a composition according to the invention may comprise at least one non-volatile hydrocarbon-based oil chosen from synthetic ethers containing from 10 to 40 carbon atoms, such as dicaprylyl ether, carbonates, such as dicaprylyl carbonate, fatty acid triglycerides, in particular saturated triglycerides such as caprylic/capric triglyceride, fatty alcohols which are liquid at room temperature, with a branched and/or unsaturated carbon chain containing from 12 to 26 carbon atoms, such as 2-octyldodecanol and esters of formula R₁COOR₂ in which R₁ represents a linear or branched fatty acid residue containing from 4 to 40 carbon atoms and R₂ represents a notably branched hydrocarbon-based chain containing from 4 to 40 carbon atoms, R₁ and R₂ being such that R₁ + R₂ is greater than or equal to 18, such as isoamyl laurate.

More preferentially, a composition according to the invention may comprise at least one non-volatile hydrocarbon-based oil chosen from synthetic ethers containing from 10 to 40 carbon atoms, such as dicaprylyl ether, carbonates, such as dicaprylyl carbonate, fatty acid triglycerides, in particular that are saturated, such as caprylic/capric triglyceride, and mixtures thereof.

In particular, such non-volatile hydrocarbon-based oils may be present in a composition according to the invention in a content ranging from 6.0% to 25.0% by weight and preferably from 10.0% to 20.0% by weight relative to the total weight of the composition.

The other fatty substances that may be present in the oily phase are, for example, fatty acids including from 8 to 30 carbon atoms, for instance stearic acid, lauric acid, palmitic acid and oleic acid; waxes, different from glyceryl trihydroxystearate, for instance lanolin, beeswax, carnauba wax or candelilla wax, paraffin wax, lignite wax or microcrystalline waxes, ceresin or ozokerite, and synthetic waxes, for instance polyethylene waxes and Fischer-Tropsch waxes; silicone resins such as trifluoromethyl-C₁-C₄-alkyl dimethicone and trifluoropropyl dimethicone; and silicone elastomers, for instance the products sold under the name KSG by the company Shin-Etsu, under the name Trefil or BY29 by the company Dow Corning, or under the name Gransil by the company Grant Industries.

As fatty substances, a composition according to the invention may preferably comprise at least one fatty alcohol wax.

Such waxes may be chosen from lauryl alcohol, myristyl alcohol, cetyl alcohol, stearyl alcohol, arachidyl alcohol, behenyl alcohol, lignoceryl alcohol, ceryl alcohol, montanyl alcohol, myricyl alcohol, and mixtures thereof.

Preferentially, the fatty alcohol wax is cetyl alcohol.

As fatty substance, a composition according to the invention may preferably comprise at least one butter, in particular a plant butter.

The plant butter(s) that are suitable for use in the invention are preferably chosen from the group comprising avocado butter, cocoa butter, shea butter, kokum butter, mango butter, murumuru butter, coconut butter, apricot kernel butter, sal butter and urukum butter, and mixtures thereof, and in particular is shea butter.

These fatty substances may be chosen in a varied manner by a person skilled in the art in order to prepare a composition having the desired properties, for example in terms of consistency or texture.

According to a preferred embodiment, a composition according to the invention comprises at least one oil chosen from mixtures of volatile linear alkanes comprising from 8 to 16 atoms, mixtures of linear and/or branched C₁₅-C₁₉ alkanes, synthetic ethers containing from 10 to 40 carbon atoms, fatty acid triglycerides, and mixtures thereof.

Preferably, a composition according to the invention comprises at least one oil chosen from mixtures of linear and/or branched C₁₅-C₁₉ alkanes, synthetic ethers containing from 10 to 40 carbon atoms, in particular dicaprylyl ether, and mixtures thereof.

Such oils are in particular present in a composition according to the invention in a content ranging from 5.0% to 30% by weight and preferably from 10% to 20% by weight relative to the total weight of the composition.

Preferably, the fatty phase content is between 5.0% and 50% by weight, in particular between 10% and 30% by weight, and preferably between 15% and 25% by weight, relative to the total weight of the composition.

### Aqueous phase

As mentioned previously, a composition according to the invention comprises at least one aqueous phase in a concentration of at least 50.0% by weight, relative to the total weight of the composition, and dispersed in the fatty phase defined above.

The aqueous phase comprises water and optionally a water-soluble solvent.

According to the present invention, the term "water-soluble solvent" denotes a compound that is liquid at room temperature and water-miscible (miscibility with water of greater than 50% by weight at 25°C and atmospheric pressure).

The water used may be demineralized water and/or a floral water such as rose water, cornflower water, chamomile water or lime blossom water, and/or a natural spring water or mineral water, for instance: Vittel water, Vichy basin water, Uriage water, Roche Posay water, Bourboule water, Enghien-les-Bains water, Saint Gervais-les-Bains water, Néris-les-Bains water, Allevar-les-Bains water, Digne water, Maizières water, Neyrac-les-Bains water, Lons-le-Saunier water, Eaux Bonnes water, Rochefort water, Saint Christau water, Fumades water, Tercis-les-Bains water and Avene water. The aqueous phase may also comprise reconstituted spring water, i.e. a water containing trace elements such as zinc, copper, magnesium, etc., reconstituting the characteristics of a spring water.

The water-soluble solvents that can be used in the composition of the invention may also be volatile.

Among the water-soluble solvents that may be used in the composition according to the invention, mention may be made notably of lower monoalcohols containing from 1 to 5 carbon atoms such as ethanol and isopropanol, glycols containing from 2 to 8 carbon atoms such as ethylene glycol, hexylene glycol, propylene glycol, 1,3-butylene glycol, pentylene glycol and dipropylene glycol, C₃ and C₄ ketones and C₂-C₄ aldehydes.

According to an embodiment variant, the aqueous phase of a composition according to the invention may comprise at least one C₂-C₃₂ polyol.

For the purposes of the present invention, the term "polyol" should be understood as meaning any organic molecule including at least two free hydroxyl groups.

Such polyols are more particularly known for giving the composition increased properties in terms of moisturization. Specifically, these compounds are capable of penetrating into the stratum corneum and of keeping it moisturized.

Preferably, a polyol in accordance with the present invention is present in liquid form at room temperature.

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are suitable for formulating a composition according to the present invention are in particular those notably containing from 2 to 32 carbon atoms, preferably from 3 to 16 carbon atoms.

The polyol may be chosen, for example, from ethylene glycol, pentaerythritol, trimethylolpropane, propylene glycol, 1,3-propanediol, butylene glycol, isoprene glycol, pentylene glycol, hexylene glycol, caprylyl glycol, glycerol, polyglycerols, such as glycerol oligomers, for instance diglycerol, polyethylene glycols, and mixtures thereof.

According to a preferred embodiment of the invention, said polyol is chosen from ethylene glycol, pentaerythritol, trimethylolpropane, propylene glycol, 1,3-propanediol, dipropylene glycol, pentylene glycol, glycerol, polyglycerols and polyethylene glycols, and mixtures thereof.

According to a particular embodiment, the composition of the invention may comprise at least one polyol, in particular chosen from glycerol, propylene glycol, pentylene glycol, 1,3-propanediol, and mixtures thereof, in particular a mixture of glycerol and at least one chosen from propylene glycol, 1,3-propanediol and pentylene glycol.

When they are present, the polyol(s) are preferably present in a composition according to the invention in a content ranging from 1.0% to 20% by weight, better still from 3.0% to 15% by weight, preferably from 5.0% to 10% by weight, relative to the total weight of said composition.

The aqueous phase may also comprise stabilizers, for example sodium chloride, magnesium dichloride or magnesium sulfate.

The aqueous phase may also comprise any water-soluble or water-dispersible compound that is compatible with an aqueous phase, such as gelling agents, film-forming polymers, thickeners or surfactants, and mixtures thereof.

Preferably, the aqueous phase is present in a composition according to the invention in a content ranging from 50% to 95% by weight, preferably from 50% to 85% by weight, and more preferentially from 50% to 75% by weight, relative to the total weight of said composition.

### Additives

A composition according to the invention may also comprise at least one additive, notably chosen from the additives detailed below.

Needless to say, the additive(s) that may be present in a composition according to the invention are chosen so as not to alter the properties of the composition, in particular in terms of stability, but also in terms of sensory properties.

Preferably, the additives that are suitable for use in the present invention are natural or of natural origin.

### Active agents

Advantageously, a composition according to the invention may also comprise at least one cosmetic active agent.

In particular, the cosmetic active agent may be at least one hydrophilic active agent.

The term "hydrophilic active agent" means a water-soluble or water-dispersible active agent which is capable of forming hydrogen bonds.

As hydrophilic active agents, examples that may be mentioned include moisturizers, depigmenting agents, desquamating agents, humectants, anti-ageing agents; mattifying agents, cicatrizing agents, antibacterial agents, and mixtures thereof.

The additional active agent(s) may notably be chosen from:
- vitamins and derivatives thereof, notably esters thereof, such as niacinamide (3-pyridinecarboxamide), nicotinamide (vitamin B3), tocopherol (vitamin E) and esters thereof (for instance tocopheryl acetate), ascorbic acid and derivatives thereof (vitamin C), retinol (vitamin A),
- humectants or moisturizers such as urea, hydroxyureas, glycerol, polyglycerols, glyceryl glucoside, diglyceryl glucoside, polyglyceryl glucosides, xylityl glucoside and plant extracts (notably of tea, mint, orchid, soybean, aloe vera, honey), and in particular glycerol;
- C-glycoside compounds, and preferably hydroxypropyl tetrahydropyrantriol (or proxylane);
- antioxidant compounds;
- anti-ageing active agents, such as hyaluronic acid compounds, and notably sodium hyaluronate, salicylic acid compounds and in particular 5-n-octanoylsalicylic acid (capryloylsalicylic acid), adenosine, C-β-D-xylopyranoside-2-hydroxypropane and the sodium salt of (3-hydroxy-2-pentylcyclopentyl)acetic acid;
- keratolytic agents such as lactic acid or glycolic acid; and
- mixtures thereof.

### C-Glycoside compounds

In particular, a composition according to the invention may comprise at least one cosmetic active agent chosen from C-glycoside compounds, in particular having the following general formula: 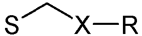 in which:
- R denotes an unsubstituted linear C₁-C₄ and notably C₁-C₂ alkyl radical, in particular methyl;
- S represents a monosaccharide chosen from D-glucose, D-xylose, N-acetyl-D-glucosamine and L-fucose, and in particular D-xylose;
- X represents a group chosen from -CO-, -CH(OH)- and -CH(NH₂)- and preferentially a -CH(OH)- group;
and also the cosmetically acceptable salts thereof, solvates thereof such as hydrates, and optical isomers thereof.

As non-limiting illustrations of C-glycosides that are more particularly suitable for use in the invention, mention may notably be made of the following compounds:
- C-β-D-xylopyranoside-n-propan-2-one;
- C-α-D-xylopyranoside-n-propan-2-one;
- C-β-D-xylopyranoside-2-hydroxypropane;
- C-α-D-xylopyranoside-2-hydroxypropane;
- 1-(C-β-D-glucopyranosyl)-2-hydroxypropane;
- 1-(C-α-D-glucopyranosyl)-2-hydroxypropane;
- 1-(C-β-D-glucopyranosyl)-2-aminopropane;
- 1-(C-α-D-glucopyranosyl)-2-aminopropane;
- 3'-(acetamido-C-β-D-glucopyranosyl)propan-2'-one;
- 3'-(acetamido-C-α-D-glucopyranosyl)propan-2'-one;
- 1-(acetamido-C-β-D-glucopyranosyl)-2-hydroxypropane;
- 1-(acetamido-C-β-D-glucopyranosyl)-2-aminopropane;
and also the cosmetically acceptable salts thereof, solvates thereof such as hydrates, and optical isomers thereof.

According to a particular embodiment, use is made of C-β-D-xylopyranoside-2-hydroxypropane or C-α-D-xylopyranoside-2-hydroxypropane, and better still C-β-D-xylopyranoside-2-hydroxypropane (or proxylane).

According to a particular embodiment, a C-glycoside of the formula illustrated above that is suitable for use in the invention may preferably be C-β-D-xylopyranoside-2-hydroxypropane, the INCI name of which is hydroxypropyl tetrahydropyrantriol, notably sold under the name Mexoryl SBB^{®}, Mexoryl SCN^{®} or Mexoryl SCS^{®} by Novéal.

The C-glycoside salts that are suitable for use in the invention may comprise conventional physiologically acceptable salts of these compounds, such as those formed from organic or inorganic acids.

Examples that may be mentioned include the salts of mineral acids, such as sulfuric acid, hydrochloric acid, hydrobromic acid, hydriodic acid, phosphoric acid and boric acid. Mention may also be made of the salts of organic acids, which may include one or more carboxylic, sulfonic or phosphonic acid groups. They may be linear, branched or cyclic aliphatic acids, or alternatively aromatic acids. These acids may also include one or more heteroatoms chosen from O and N, for example in the form of hydroxyl groups. Mention may notably be made of propionic acid, acetic acid, terephthalic acid, citric acid and tartaric acid.

The solvates that are acceptable for the compounds described above comprise conventional solvates such as those formed during the final step of preparation of said compounds due to the presence of solvents. Examples that may be mentioned include solvates due to the presence of water or of linear or branched alcohols, such as ethanol or isopropanol.

The C-glycosides having the formula illustrated above are known from WO 02/051828.

According to one embodiment, the composition according to the invention comprises a C-glycoside in an amount of between 0.1% and 15% by weight relative to the total weight of the composition, in particular between 0.5% and 10% by weight relative to the total weight of the composition and more particularly between 1.0% and 5.0% by weight relative to the total weight of the composition.

### Niacinamide compound

In particular, a composition according to the invention may comprise at least one cosmetic active chosen from niacinamide compounds, in particular chosen from niacinamide (also known as vitamin B3), N,N-diethylniacinamide, N-picolylniacinamide and N-allylniacinamide. According to a preferred embodiment, the niacinamide compound is niacinamide.

A composition according to the invention may comprise from 0.1% to 10% by weight and preferably from 0.5% to 5% by weight of adenosine or analogue relative to the total weight of the composition.

### Adenosine and analogues thereof

In particular, a composition according to the invention may comprise an cosmetic active agent chosen from adenosine and analogues thereof.

Among the adenosine analogues that may be used according to the invention, mention will be made notably of adenosine receptor agonists and compounds that increase the intracellular or extracellular levels of adenosine.

Examples of adenosine analogues include 2'-deoxyadenosine; 2',3'-isopropylideneadenosine, toyocamycin, 1-methyladenosine; N-6-methyladenosine; adenosine N-oxide, 6-methylmercaptopurine riboside; 6-chloropurine riboside, 5'-adenosine monophosphate; 5'-adenosine diphosphate and 5'-adenosine triphosphate, phenylisopropyladenosine ("PIA"), 1-methylisoguanosine, N-6-cyclohexyladenosine (CHA), N-6-cyclopentyladenosine (CPA), 2-chloro-N-6-cyclopentyladenosine, 2-chloroadenosine, N-6-phenyladenosine, 2-phenylaminoadenosine, N-6-phenethyladenosine, 2-p-(2-carboxyethyl)phenethylamino-5'-N-ethylcarboxamidoadenosine (CGS-21680), N-10-ethylcarboxamidoadenosine (NECA) 5'-(N-cyclopropyl)carboxamidoadenosine, metrifudil, erythro-9-(2-hydroxy-3-nonyl)adenine ("EHNA") and iodotubercidine.

Adenosine is preferred for use in the present invention. It is notably commercially available in powder form from the company Pharma Waldhof.

A composition according to the invention may comprise from 0.001% to 10% by weight and preferably from 0.01% to 1% by weight of adenosine or analogue relative to the total weight of the composition.

### Ascorbic acid and derivatives

In particular, a composition according to the invention may comprise at least one cosmetic active agent chosen from ascorbic acid, also called vitamin C, and/or a derivative thereof. The ascorbic acid may be in D or L form, advantageously in L form, and analogues thereof chosen from salts thereof, preferably sodium ascorbate, magnesium or sodium ascorbyl phosphate, and glycosyl ascorbic acid.

Ascorbic acid derivatives that may be mentioned in particular include sugar esters of ascorbic acid and metal salts of phosphorylated ascorbic acid.

The sugar esters of ascorbic acid that may be used in the invention are notably glycosyl, mannosyl, fructosyl, fucosyl, galactosyl, N-acetylglucosamine and N-acetylmuramic derivatives of ascorbic acid and mixtures thereof and more notably ascorbyl-2 glucoside or 2-O-α-D-glucopyranosyl L-ascorbic acid or else 6-O-β-D-galactopyranosyl L-ascorbic acid.

The latter compounds and processes for preparing them are described in particular in EP 487 404, EP 425 066 and J05213736.

For its part, the metal salt of phosphorylated ascorbic acid is chosen from alkali metal ascorbyl phosphates, alkaline-earth metal ascorbyl phosphates and transition metal ascorbyl phosphates.

The ascorbic acid derivatives that are suitable for use according to the present invention may be chosen from the 5,6-di-O-dimethylsilyl ascorbate sold under the reference PRO-AA by the company Exsymol, the potassium salt of dl-α-tocopheryl-dl-ascorbyl phosphate sold under the reference Sepivital EPC by the company Senju Pharmaceutical, magnesium ascorbyl phosphate, sodium ascorbyl phosphate sold under the reference Stay-C 50 by the company Roche and ascorbyl glucoside sold by the company Hayashibara.

Magnesium ascorbyl phosphate is preferably used.

A composition according to the invention may in particular comprise from 0.1% to 8% by weight of ascorbic acid and/or a derivative thereof, in particular from 0.5% to 5% by weight, preferably from 1% to 3% by weight relative to the total weight of the composition.

### Hyaluronic acid

In particular, a composition according to the invention may comprise at least one cosmetic active agent chosen from hyaluronic acid or a derivative thereof.

In the context of the present invention, the term "hyaluronic acid or a derivative thereof" notably covers the hyaluronic acid basic unit having the formula:

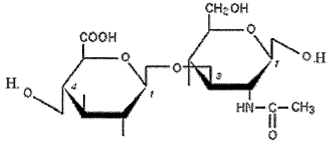

This is the smallest fraction of hyaluronic acid, comprising a disaccharide dimer, namely D-glucuronic acid and N-acetylglucosamine.

In the context of the present invention, the term "hyaluronic acid or a derivative thereof" also comprises the linear polymer comprising the polymeric unit described above, according to a sequence with alternating β(1,4) and β(1,3) glycosidic bonds, having a molecular weight (MW) which may range between 380 and 13 000 000 daltons. This molecular weight mainly depends on the source from which the hyaluronic acid is obtained and/or the preparation methods.

In the context of the present invention, the term "hyaluronic acid or a derivative thereof" also comprises hyaluronic acid salts and notably alkali metal salts such as the sodium salt and the potassium salt.

In the natural state, hyaluronic acid is present in pericellular gels, in the ground substance of connective tissues of vertebrate organs such as the dermis and epithelial tissues and in particular in the epidermis, in the articular synovial fluid, in the vitreous humour, in the human umbilical cord and in the *crista galli* apophysis.

Thus, the term "hyaluronic acid or a derivative thereof" comprises all of the hyaluronic acid fractions or subunits having a molecular weight notably within the molecular weight range recalled above.

In the context of the present invention, it is preferred to use hyaluronic acid fractions not having any inflammatory activity.

As illustrations of the various hyaluronic acid fractions, reference may be made to the document Hyaluronan fragments: an information-rich system, R. Stern et al., European Journal of Cell Biology 58 (2006) 699-715, which reviews the listed biological activities of hyaluronic acid as a function of its molecular weight.

According to a favoured embodiment of the invention, the hyaluronic acid fractions that are suitable for the application intended by the present invention have a molecular weight of between 50 000 and 5 000 000, in particular between 100 000 and 5 000 000, notably between 400 000 and 5 000 000 Da. It is a matter in this case of high molecular weight hyaluronic acid.

Alternatively, the hyaluronic acid fractions that may also be suitable for the application intended by the present invention have a molecular weight of between 50 000 and 400 000 Da. It is a matter in this case of intermediate molecular weight hyaluronic acid.

Alternatively also, the hyaluronic acid fractions that may be suitable for the application intended by the present invention have a molecular weight of less than 50 000 Da. It is a matter in this case of low molecular weight hyaluronic acid.

Finally, the term "hyaluronic acid or a derivative thereof" also comprises hyaluronic acid esters, notably those in which all or some of the carboxylic groups of the acid functions are esterified with oxyethylenated alcohols or alkyls, including from 1 to 20 carbon atoms, notably with a degree of substitution on the D-glucuronic acid of the hyaluronic acid ranging from 0.5% to 50%.

Mention may notably be made of the methyl, ethyl, n-propyl, n-pentyl, benzyl and dodecyl esters of hyaluronic acid. Such esters have notably been described in D. Campoccia et al. "Semisynthetic resorbable materials from hyaluronan esterification", Biomaterials 19 (1998) 2101-2127.

The molecular weights indicated above are also valid for the hyaluronic acid esters.

Hyaluronic acid may notably be sold by the company Hyactive under the trade name CPN (MW: 10 to 150 kDa), by the company Soliance under the trade name Cristalhyal (MW: 1.1×10⁶), by the company Bioland under the name Nutra HA (MW: 820 000 Da), by the company Bioland under the name Nutra AF (MW: 69 000 Da), by the company Bioland under the name Oligo HA (MW: 6100 Da) or by the company Vam Farma Cosmetica under the name D Factor (MW: 380 Da).

In one embodiment, the hyaluronic acid is in the form of spheres. In particular, such spheres are sold by the company BASF under the name Hyaluronic Acid Spheres. This is a mixture of hyaluronic acid of different molecular weights, namely of MW 1.5 x 10⁶, 400 000 and 600 000 Da.

Sodium hyaluronate is preferably used.

The hyaluronic acid or a derivative thereof is present in the composition according to the present invention in a content of between 0.01% and 5%, preferably between 0.1% and 3% and more particularly between 0.2% and 1% by weight relative to the total weight of the composition.

### Salicylic acid compounds

In particular, a composition according to the invention may comprise at least one cosmetic active agent chosen from salicylic acid compounds.

The salicylic acid compound present in the composition according to the invention is preferably chosen from salicylic acid and compounds having the following formula: 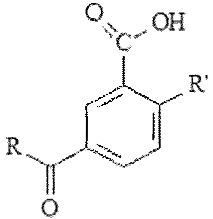 in which:
- the radical R denotes a linear, branched or cyclic saturated aliphatic chain containing from 2 to 22 carbon atoms; an unsaturated chain containing from 2 to 22 carbon atoms, containing one or more double bonds that may be conjugated; an aromatic nucleus linked to the carbonyl radical directly or via saturated or unsaturated aliphatic chains containing from 2 to 7 carbon atoms; said groups possibly being substituted with one or more substituents, which may be identical or different, chosen from (a) halogen atoms, (b) the trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms;
- R' is a hydroxyl group;
and also salts thereof derived from a mineral or organic base.

Preferentially, the radical R denotes a linear, branched or cyclic saturated aliphatic chain containing from 3 to 11 carbon atoms; an unsaturated chain containing from 3 to 17 carbon atoms and comprising one or more conjugated or unconjugated double bonds; said hydrocarbon-based chains possibly being substituted with one or more substituents, which may be identical or different, chosen from (a) halogen atoms, (b) the trifluoromethyl group, (c) hydroxyl groups in free form or esterified with an acid containing from 1 to 6 carbon atoms, or (d) a carboxyl function in free form or esterified with a lower alcohol containing from 1 to 6 carbon atoms; and salts thereof obtained by salification with a mineral or organic base.

The compounds that are more particularly preferred are those in which the radical R is a C₃-C₁₁ alkyl group.

Among the salicylic acid compounds that are particularly preferred, mention may be made of 5-n-octanoylsalicylic acid (or capryloylsalicylic acid); 5-n-decanoylsalicylic acid; 5-n-dodecanoylsalicylic acid; 5-n-heptanoylsalicylic acid, and the corresponding salts thereof.

The salicylic acid compound is preferably chosen from salicylic acid and 5-n-octanoylsalicylic acid, and more preferentially is 5-n-octanoylsalicylic acid.

The salts of the compounds having the formula illustrated above may be obtained by salification with a mineral or organic base. Examples of mineral bases that may be mentioned include alkali metal or alkaline-earth metal hydroxides, for instance sodium hydroxide or potassium hydroxide, or ammonia.

Among the organic bases, mention may be made of amines and alkanolamines. Quaternary salts, for instance those described in the patent FR 2 607 498, are particularly advantageous. The compounds having the formula illustrated above that may be used according to the invention are described in patents US 6 159 479 and US 5 558 871, FR 2 581 542, FR 2 607 498, US 4 767 750, EP 378 936, US 5 267 407, US 5 667 789, US 5 580 549 and EP-A-570 230.

According to one embodiment, a composition according to the invention may comprise at least one salicylic acid compound in an amount of between 0.05% and 5% by weight relative to the total weight of the composition, in particular between 0.1% and 2% by weight and more particularly between 0.2% and 1% by weight.

### Plant extract

In particular, a composition according to the invention may comprise at least one plant extract, in particular an *Aloe vera* extract.

The *Aloe vera* extract may be present in a composition according to the invention in a content of at least 0.01% by weight and at most 1% by weight, preferably from 0.05% to 0.4% by weight relative to the total weight of the composition.

The material preferentially used is known under the trade reference Aloe Vera Freeze-Dried Powder 200:1 from Mexi Aloe Lab.

### Retinol

In particular, a composition according to the invention may comprise retinol, otherwise known as vitamin A.

For the purposes of the present invention, the term *"retinol"* is intended to denote all isomers of retinol, notably all-trans retinol, 13-cis retinol, 11-cis retinol, 9-cis retinol and 3,4-didehydroretinol.

Preferably, all-trans retinol is used.

A composition according to the invention may comprise an amount of retinol of between 0.02% and 5.0% by weight, notably between 0.05% and 3.0% by weight, preferably between 0.08% and 1.0% by weight and more preferentially between 0.1% and 0.5% by weight relative to the total weight of the composition.

It is understood that the retinol content corresponds to the content of active material, also known as the solids content, of retinol introduced into the composition.

According to a particular embodiment variant, retinol can be introduced into the composition dissolved in an oil, such as a plant oil, for example soya oil, notably in a content ranging from 5% to 20% by weight, preferably about 10% by weight in the oil.

The products sold by the company BASF notably under the name Retinol 10SU, at 10% by weight of active material in soybean oil, are most particularly suitable for use.

According to another particular embodiment variant, an encapsulated form of retinol may also be used.

Advantageously, a composition according to the invention may comprise at least one cosmetic active agent chosen from humectants, preferably glycerol; C-glycoside compounds, and preferably hydroxypropyl tetrahydropyrantriol; hyaluronic acid compounds, and notably sodium hyaluronate, salicylic acid compounds, and in particular 5-n-octanoylsalicylic acid (capryloylsalicylic acid), ascorbic acid and derivatives thereof, lactic acid, adenosine and analogues thereof, plant extracts, retinoids, in particular retinol, niacinamide compounds; and mixtures thereof.

Even more preferably, a composition according to the invention may comprise at least one anti-ageing cosmetic active agent, in particular chosen from hydroxypropyltetrahydropyrantriol, or proxylane, adenosine, niacinamide compounds, salicylic acid compounds, ascorbic acid, and mixtures thereof, more particularly chosen from hydroxypropyltetrahydropyrantriol, adenosine, niacinamide compounds, and mixtures thereof.

A composition according to the invention may comprise from 0.5% to 10% by weight of active agent(s), in particular of anti-ageing active agent(s), preferably from 1% to 8% by weight of active agent(s), relative to the total weight of the composition.

According to one embodiment, a composition according to the invention comprises at least one cosmetic active agent chosen from C-glycoside compounds.

Thus, according to one of its aspects, the present invention relates to a composition, notably a cosmetic composition, in particular for making up and/or caring for keratin materials, comprising:
- at least one continuous fatty phase;
- at least one aqueous phase in a concentration of at least 50.0% by weight, relative to the total weight of the composition, and dispersed in said fatty phase;
- at least two esters that are different from each other, chosen from esters of polyglycerol with at least one fatty acid containing from 8 to 30 carbon atoms;
- glyceryl trihydroxystearate;
- at least 6.0% by weight, relative to the total weight of the composition, of at least one filler chosen from talc, hydrophobic silica aerogel particles, C₈-C₂₂ N-acylamino acid particles, modified or unmodified starches, boron nitride, polymeric fillers, metal oxides, and mixtures thereof; and
- at least one C-glycoside compound, preferably chosen from C-β-D-xylopyranoside-2-hydroxypropane or C-α-D-xylopyranoside-2-hydroxypropane, and better still C-β-D-xylopyranoside-2-hydroxypropane (or proxylane).

### Surfactants

A composition according to the invention may comprise emulsifying surfactants, which are preferably nonionic, different from the polyglycerol esters defined above.

As examples of emulsifying surfactants, mention may be made of esters or ethers of sorbitan, of polyols or of sugars, other than polyglycerols.

As examples of polyol alkyl esters, mention may be made of polyethylene glycol esters, for instance PEG-30 dipolyhydroxystearate, such as the product sold under the name Arlacel^{®} P135 by the company ICI.

As examples of glycerol and/or sorbitan esters, mention may be made of sorbitan isostearate, such as the product sold under the name Arlacel^{®} 987 by the company ICI or sorbitan glyceryl isostearate, such as the product sold under the name Arlacel^{®} 986 by the company ICI, and mixtures thereof.

As examples of emulsifying surfactants, mention may be made of silicone surfactants, such as dimethicone copolyols or silicone elastomers.

As examples of dimethicone copolyols, mention may be made of the product having the INCI name Dimethicone (and) PEG/PPG-18/18 Dimethicone sold under the brand X-22-6711D^{®} by the company Shin-Etsu, the mixture of cyclomethicone and dimethicone copolyol, sold under the name DC5225^{®} C by the company Dow Corning, and alkyl dimethicone copolyols such as the lauryl methicone copolyol sold under the name DC5200 Formulation Aid by the company Dow Corning; cetyl dimethicone copolyol, for instance cetyl PEG/PPG-10/1 Dimethicone such as the product sold under the name Abil EM^{®} 90 by the company Evonik Goldschmidt.

Advantageously, a composition according to the invention may comprise at least one emulsifying surfactant chosen from C₈-C₂₄, in particular C₁₂-C₂₂, fatty acid esters of glycerol, such as the glyceryl ester of stearic acid (name CTFA: Glyceryl Stearate).

A composition according to the invention may comprise between 0.01% and 2.0% by weight of emulsifying surfactant, different from the polyglycerol esters defined above, in particular a glycerol ester of stearic acid, preferably between 0.05% and 1.5% by weight, more preferentially between 0.1% and 1.0% by weight, relative to the total weight of the composition.

### Dyestuffs

A composition according to the invention may also comprise at least one particulate or non-particulate, water-soluble or water-insoluble dyestuff, preferably in a proportion of at least 0.01% by weight relative to the total weight of the composition.

For obvious reasons, this amount is liable to vary significantly with regard to the intensity of the desired colour effect and of the colour intensity afforded by the dyestuffs under consideration, and its adjustment clearly falls within the competence of a person skilled in the art.

A composition according to the invention may comprise from 0.01% to 25% by weight, notably from 0.1% to 25% by weight, in particular from 1.0% to 20% by weight and preferably from 2.5% to 15% by weight of dyestuffs relative to the total weight of said composition.

As stated above, the dyestuffs that are suitable for use in the invention may be water-soluble, but may also be liposoluble.

For the purposes of the invention, the term "water-soluble dyestuff" means any natural or synthetic, generally organic compound, which is soluble in an aqueous phase or water-miscible solvents and which is capable of imparting colour.

As water-soluble dyes that are suitable for use in the invention, mention may be made notably of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophyllin, methylene blue, anthocyanins (enocianin, black carrot, hibiscus, elder), caramel and riboflavin.

The water-soluble dyes are, for example, beetroot juice and caramel.

For the purposes of the invention, the term "liposoluble dyestuff" means any natural or synthetic, generally organic compound, which is soluble in an oily phase or in solvents that are miscible with a fatty substance, and which is capable of imparting colour.

As liposoluble dyes that are suitable for use in the invention, mention may notably be made of synthetic or natural liposoluble dyes, for instance DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto and curcumin.

The colouring particulate materials may be present in a proportion of from 0.01% to 25% by weight relative to the total weight of the composition containing them.

They may notably be pigments, nacres and/or particles with metallic tints.

The term "pigments" should be understood as meaning white or coloured, mineral or organic particles that are insoluble in an aqueous solution, which are intended to colour and/or opacify the composition containing them.

A composition according to the invention may comprise from 0.01% to 25% by weight, notably from 0.1% to 25% by weight, in particular from 1.0% to 25% by weight and preferably from 2.5% to 15% by weight of pigments relative to the total weight of said composition.

Preferably, when the composition according to the invention is a makeup composition, it may comprise at least 2.5% by weight, preferably at least 10% by weight and more preferentially at least 15% by weight, of pigments relative to the total weight of said composition.

The pigments may be white or coloured, and mineral and/or organic.

As mineral pigments that may be used in the invention, mention may be made of titanium oxide, titanium dioxide, zirconium oxide, zirconium dioxide, cerium oxide or cerium dioxide and also zinc oxide, iron oxide or chromium oxide, ferric blue, manganese violet, ultramarine blue and chromium hydrate, and mixtures thereof.

They may also be pigments having a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

They may also be pigments having a structure that may be, for example, of silica microsphere type containing iron oxide. An example of a pigment having this structure is the product sold by Miyoshi under the reference PC Ball PC-LL-100 P, this pigment being constituted of silica microspheres containing yellow iron oxide.

Advantageously, the pigments in accordance with the invention are iron oxides and/or titanium dioxides.

The term"nacres" should be understood as meaning iridescent or non-iridescent coloured particles of any shape, notably produced by certain molluscs in their shell, or alternatively synthesized, which have a colour effect via optical interference.

A composition according to the invention may comprise from 0% to 15% by weight of nacres relative to the total weight of said composition.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic dyestuffs.

Examples of nacres that may also be mentioned include natural mica covered with titanium oxide, with iron oxide, with natural pigment or with bismuth oxychloride.

Among the nacres available on the market, mention may be made of the Timica, Flamenco and Duochrome nacres (based on mica) sold by the company Engelhard, the Timiron nacres sold by the company Merck, the Prestige mica-based nacres, sold by the company Eckart, and the Sunshine synthetic mica-based nacres, sold by the company Sun Chemical.

The nacres may more particularly have a yellow, pink, red, bronze, orange, brown, gold and/or coppery colour or tint.

Advantageously, the nacres in accordance with the invention are micas covered with titanium dioxide or with iron oxide, and also bismuth oxychloride.

For the purposes of the present invention, the term "particles with a metallic tint" means any compound whose nature, size, structure and surface finish allow it to reflect the incident light, notably in a non-iridescent manner.

The particles with a metallic tint that may be used in the invention are chosen in particular from:
- particles of at least one metal and/or of at least one metal derivative;
- particles including a single-material or multi-material organic or mineral substrate, at least partially coated with at least one layer with a metallic tint comprising at least one metal and/or at least one metal derivative; and
- mixtures of said particles.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr, and mixtures or alloys thereof (for example bronzes and brasses) are preferred metals.

The term "metal derivatives" denotes compounds derived from metals, in particular oxides, fluorides, chlorides and sulfides.

Illustrations of these particles that may be mentioned include aluminium particles, such as those sold under the names Starbrite 1200 EAC^{®} by the company Siberline and Metalure^{®} by the company Eckart and glass particles coated with a metallic layer, notably those described in JP-A-09188830, JP-A-10158450, JP-A-10158541, JP-A-07258460 and JP-A-05017710.

### Hydrophobic treatment of the colorants

The pulverulent dyestuffs as described previously may be totally or partially surface treated, with a hydrophobic agent, to make them more compatible with the oily phase of the composition of the invention, notably so that they have good wettability with oils. Thus, these treated pigments are well dispersed in the oily phase.

Hydrophobically treated pigments are notably described in EP-A-1 086 683.

The hydrophobic-treatment agent may be chosen from silicones such as methicones, dimethicones and perfluoroalkylsilanes; fatty acids, such as stearic acid; metal soaps, such as aluminium dimyristate, the aluminium salt of hydrogenated tallow glutamate; perfluoroalkyl phosphates; polyhexafluoropropylene oxides; perfluoropolyethers; amino acids; N-acylamino acids or salts thereof; lecithin, isopropyl triisostearyl titanate, isostearyl sebacate, and mixtures thereof.

The term "alkyl" mentioned in the compounds cited previously notably denotes an alkyl group containing from 1 to 30 carbon atoms and preferably containing from 5 to 16 carbon atoms.

### Additional fillers

A composition according to the invention may also comprise one or more additional fillers, different from the fillers used according to the invention and detailed above.

As illustrations of these additional fillers, mention may be made of mica, silica, kaolin, hollow silica microspheres, celluloses and mixtures thereof.

Preferably, a composition according to the invention comprises less than 2% by weight, preferably less than 1% by weight, and more preferentially is free of additional filler(s) different from the fillers required according to the invention.

### Adjuvants

A composition according to the invention may also include at least one adjuvant that is customary in the cosmetic field, chosen from fragrances, film-forming polymers, pH adjusters (acids or bases), for example citric acid, tartaric acid or oxalic acid, chelating agents, preserving agents, and mixtures thereof.

Needless to say, a person skilled in the art will take care to select this or these optional additional compound(s), and/or the amount thereof, such that the advantageous properties of a composition according to the invention are not, or are not substantially, adversely affected by the envisioned addition.

Advantageously, a composition according to the invention may comprise at least one preserving agent, preferably chosen from pentylene glycol, salicylic acid, potassium sorbate, phytic acid, and mixtures thereof, in particular in a content ranging from 1.0% to 12% by weight, preferably from 2.0% to 9.0%, more preferentially from 4.0% to 9.0% by weight, relative to the total weight of the composition.

As mentioned above, a composition according to the invention is preferably free of compounds that may be harmful to man and/or the environment, i.e. it comprises less than 0.01% by weight, or is even free of compounds that may be harmful to man and/or the environment.

Thus, a composition according to the invention is in particular free of silicone compounds and/or of ethylenediaminetetraacetic acid (EDTA), and is preferably free of silicone compounds and of ethylenediaminetetraacetic acid.

### Intended use of the composition

A composition according to the invention may be in the form of a cosmetic composition for caring for and/or making up keratin materials, preferably a cosmetic composition for caring for keratin materials, in particular of the body or of the face, preferably of the face.

These compositions may constitute cleansing, protective, treating or care creams for the face, the hands or the body, for example day creams, night creams, makeup creams, foundation creams or antisun creams.

According to one embodiment, a composition according to the invention is in the form of a composition for caring for keratin materials, in particular the skin of the body or the face, preferably of the face.

In particular, a composition of the invention may be in the form of an anti-ageing, moisturising or photoprotective care composition, in particular an anti-ageing care composition, for the skin of the body or of the face, in particular of the face.

According to another embodiment, a composition of the invention may be in the form of a composition for making up keratin materials, in particular of the body or of the face, preferably of the face.

Thus, according to a submode of this embodiment, a composition of the invention may be in the form of a makeup base composition for making up. A composition of the invention may in particular be in the form of a foundation or a tinted cream.

Such compositions are notably prepared according to the general knowledge of a person skilled in the art.

Thus, the invention also relates to the use of a composition according to the invention for caring for and/or making up keratin materials, preferably for caring for keratin materials, in particular the skin of the body and/or of the face.

The invention also relates to a cosmetic process for making up and/or caring for keratin materials, in particular the skin, comprising at least one step of applying a composition as defined previously to said keratin materials.

Preferably, the invention also relates to a cosmetic process for caring for keratin materials, in particular the skin, comprising at least one step of applying a composition as defined above to said keratin materials.

The cosmetic processes for making up and/or caring for keratin materials, in particular the skin, are non-therapeutic.

In particular, a composition according to the invention may be used for combating the signs of skin ageing.

Thus, the present patent application also relates to the use of a composition according to the invention for combating the signs of skin ageing.

The composition may be applied to the skin by hand or using an applicator.

The expressions "between... and...", "comprises from ... to...", "formed from ... to..." and "ranging from... to..." should be understood as being inclusive of the limits, unless otherwise specified.

The invention is illustrated in greater detail by the examples presented below. Unless otherwise indicated, the amounts indicated are expressed as mass percentages.

### Example

### Measurement and evaluation methods

### Stability measurement

A first method for evaluating the stability is performed by observation of the composition over time, compared to a reference.

The instability may be detected by evaluating the leaching, creaming, coalescence, formation of a film at the surface, marbling, etc.

Variations in texture, appearance, colour and odour may also be evaluated.

In particular, the compositions are observed after storage for one and/or two months at different temperatures, i.e. at 4°C, room temperature, 37°C and/or 45°C.

Another method for evaluating the stability is to observe the formulations under a microscope in non-polarized light at ×100 and if necessary ×400 magnifications.

This makes it possible to evaluate the fineness of the drops and/or the absence of aggregates and/or the presence of crystals.

The stability of the composition can be further evaluated by examining the defects in the composition after several cycles of temperature change.

Each cycle is constituted of the following steps:
a) maintaining the composition at 20°C for 6 hours;
b) decreasing the temperature to -20°C over a period of 6 hours;
c) maintaining the composition at -20°C for 6 hours; and
d) increasing the temperature to 20°C over a period of 6 hours.

Ten cycles are performed and the defects of the composition are evaluated between each cycle.

The appearance of defects between the first and fifth cycles inclusive indicates significant instability of the composition.

Finally, the stability of the composition can be evaluated using a vibrating plate test to replicate the vibratory stresses experienced by the compositions during transport. The test is performed by placing the composition, previously packed in glass jars, for example of 30 mL, on a vibrating plate rotating back and forth at 400 rpm for a period of 20 hours.

### Evaluation of the sensory properties

The sensory properties of the compositions are evaluated monadically by a panel of 20 people between 40 and 60 years old, skilled and trained in the description of care products, having applied daily a composition according to the invention or a comparative composition for 10 days.

For this purpose, the products are packaged in standard transparent 15 ml jars and coded. In the course of one session, samples are presented in random order to each assessor. 27 descriptors are evaluated in five sequences: appearance of the product in the jar, uptake, application to the hand, application to the face and 2 minutes after application. Each descriptor is rated on a 15 cm continuous scale bounded by a "Weak" reference product and a "Strong" reference product. The evaluation is then transcribed into a score from 0 to 15. In particular, the texture of the composition, the tack, the greasiness and the glidance during application and after application on the skin are determined.

The cosmetic performance of the compositions after 4 days of application is also evaluated.

### Example 1

The anti-ageing care composition for the face 1 according to the invention, in the form of a water-in-oil emulsion, and a composition 2 outside the invention, are prepared in the weight proportions as detailed in Table 1 below. The values are expressed as weight percentages relative to the total weight of the composition.

**Table 1**

| Phase | Compound (INCI name) | Formulation 1 according to the invention | Formulation 2 outside the invention |
|---|---|---|---|
| A | Dicaprylyl ether (Cetiol^{®} OE from the company BASF) | 13.00 | 12.00 |
| | C₁₅-C₁₉ Alkane (Emogreen^{®} L15 from the company Evonik) | 3.50 | 4.00 |
| | Glyceryl stearate (Tegin M Pellets^{®} from the company Evonik) | - | 0.50 |
| | Undecane (and) Tridecane (Cetiol^{®} UT from the company BASF) | - | 3.00 |
| | Polyglyceryl-4 diisostearate/polyhydroxystearate/sebacate (and) caprylic/capric triglyceride (and) glyceryl-3 oleate (and) diisostearyl polyglyceryl-3 dimer dilinoleate (Isolan^{®} 17 MB from the company Evonik) | 3.50 | 4.00 |
| | Polyglyceryl-4 isostearate (Isolan^{®} GI34 from the company Evonik) | 1.70 | 2.00 |
| | Trihydroxystearin (Thixcin^{®} R from the company Elementis) | 0.65 | 0.50 |
| B | Water | qs | qs |
| | Propanediol (1,3-Propanediol from the company Zhangjiagang Glory Chemical) | - | 3.00 |
| | Glycerin (Glycerine 4811 RSPO MB from the company Oleon) | 5.00 | 5.00 |
| | Propylene glycol (1,2-Propylene Glycol Care from the company BASF) | 0.34 | - |
| | Magnesium sulfate (Ronacare^{®} Magnesium Sulfate from the company Merck) | 1.74 | 2.00 |
| | Tocopherol (DL α Tocopherol from the company DSM) | 0.01 | 0.01 |
| | Arginine (L-Arginine C Grade from the company Ajinomoto) | - | 0.20 |
| | Potassium sorbate (Ronacare^{®} Potassium Sorbate from the company Merck) | 0.08 | 0.15 |
| | Pentylene glycol (A-Leen^{®} 5 from the company Minasolve) | 0.80 | - |
| | Salicylic acid (Salicylic Acid from the company JQC) | 0.20 | 0.20 |
| | Phytic acid (Phytic Acid 50% Solution^{®} from the company Tsuno) | 0.08 | 0.08 |
| | Adenosine (Adenosine (L) from the company Xinxiang Tuoxin Pharm) | 0.04 | - |
| | Hydroxypropyl tetrahydropyrantriol (*) (Mexoryl SCS from the company Novéal) | 4.30 | - |
| C | Lauroyl lysine (Amihope^{®} LL from the company Ajinomoto) | 10.0 | 2.00 |
| | Zea mays (corn) starch (Maisita 9060 from the company Agrana Starke) | 3.00 | - |
| D | Ethanol (Ethanol TSDA Cosmos Organic CT from the company Earth Oil) | 4.18 | 4.66 |

| | | | |
|---|---|---|---|
| (*) 70% active material | | | |

### Protocol for preparation of the compositions

The compositions are prepared according to the protocol described in detail below, in a Minilab reactor.

Place phase A in a tank and heat to a temperature of between 50°C and 55°C, then stir for 10 minutes using paddles at 30 rpm and a rotor at 4000 rpm.

In parallel, weigh out phase B and heat in a deflocculator at 50°C until totally homogenized. In the tank at 50-55°C, stop the hot water bath and add phase B through the introduction cone over 15-20 minutes with a paddle speed of 30 rpm and a rotor speed of 4000 rpm, then emulsify under vacuum at -0.5 bar for about 5 minutes using paddles at 30 rpm and a rotor at 4000 rpm.

Start cooling in a water bath at 20°C and leave to stir under vacuum at -0.5 bar using paddles at 20 rpm.

When the temperature is below 30°C, incorporate the fillers from the top of the tank with stirring using paddles at 30 rpm and then 50 rpm for 2 min to slurry the particles. Lift the lid to scrape off the excess and recommence stirring under vacuum at -0.7 bar and stirring with a rotor at 4000 rpm and paddles at 40 rpm.

Incorporate the alcohol phase through the introduction cone over about 5 minutes followed by 10 minutes of homogenization under vacuum at -0.7 bar using paddles at 40 rpm and a rotor at 4000 rpm.

### Comparison and results

The sensory properties of formulations 1 and 2 were evaluated by the panel as detailed above, compared with each other and with the commercial composition according to Table 2 below.

**Table 2**

| Composition | Trade name | Type |
|---|---|---|
| Formula 3 outside the invention | Crème de la mer^{™} *intense regenerating cream* | Water-in-oil emulsion |

Formulation 3, which is not part of the invention, differs from formulations 1 and 2 in that it is a water-in-oil emulsion.

Moreover, this formulation does not include any filler as required according to the invention.

### Results

Formulations 1 and 2 were similar in terms of texture when opened, but formulation 1, according to the invention, offered a better compromise between thickness and fluidity.

Formulation 1, according to the invention, was easy to apply to the skin, gave a fresh sensation on application and afforded good moisturization of the skin and smoothing of wrinkles for 24 hours.

A quarter of the panel felt that formulation 2, outside the invention, formed a greasy film on the skin when applied and had a shiny finish.

Formulation 3, outside the invention, was too thick and was not satisfactory in terms of softness. It was difficult to apply, uncomfortable when applied and had a greasy, tacky finish on the skin.

After 4 days of application, formulation 1, according to the invention, allowed good moisturization of the skin, reduced the visibility of wrinkles and fine lines and made the skin more supple, softer and more radiant. Half of the panel considered that the skin was smoother after 4 days of use of formulation 1 according to the invention, whereas only a quarter of the panel considered that the skin was smoother with formulation 2, not in accordance with the invention.

After 4 days of application, a quarter of the panel considered that formulation 3, outside the invention, was stiff and/or unsatisfactory in terms of skin moisturization. In addition, this formulation did not achieve the anti-ageing performance obtained with formulation 1 according to the invention.

### Example 2

The anti-ageing facial care compositions, in the form of water-in-oil emulsions, 4 and 5 according to the invention, and composition 6, outside the invention, are prepared in the weight proportions as detailed in Table 3 below. The values are expressed as weight percentages relative to the total weight of the composition.

The protocol for preparing these formulations is identical to that detailed in Example 1 above.

**Table 3**

| Phase | Compound (INCI name) | Formulation 4 according to the invention | Formulation 5 according to the invention | Formulation 6 outside the invention |
|---|---|---|---|---|
| A | Dicaprylyl ether (Cetiol^{®} OE from the company BASF) | 10.00 | 13.00 | 15.00 |
| | C₁₅-C₁₉ Alkane (Emogreen^{®} L15 from the company Evonik) | 4.00 | 3.50 | 4.00 |
| | Polyglyceryl-4 diisostearate/polyhydroxystearate/sebacate (and) caprylic/capric triglyceride (and) glyceryl-3 oleate (and) diisostearyl polyglyceryl-3 dimer dilinoleate (Isolan^{®} 17 MB from the company Evonik) | 4.00 | 3.50 | 4.00 |
| | Polyglyceryl-4 isostearate (Isolan^{®} GI34 from the company Evonik) | 2.00 | 1.70 | 2.00 |
| | Trihydroxystearin (Thixcin^{®} R from the company Elementis) | 0.75 | 0.65 | 0.75 |
| B | Water | qs | qs | qs |
| | Glycerin (Glycerine 4811 RSPO MB from the company Oleon) | 5.00 | 5.00 | 5.00 |
| | Magnesium sulfate (Ronacare^{®} Magnesium Sulfate from the company Merck) | 2.00 | 1.74 | 2.00 |
| | Tocopherol (DL α Tocopherol from the company DSM) | - | 0.01 | 0.01 |
| | Potassium sorbate (Ronacare^{®} Potassium Sorbate from the company Merck) | 0.15 | 0.08 | 0.15 |
| | Pentylene glycol (A-Leen^{®} 5 from the company Minasolve) | 0.80 | 0.80 | 0.80 |
| | Salicylic acid (Salicylic Acid from the company JQC) | 0.20 | 0.20 | 0.20 |
| | Adenosine (Adenosine (L) from the company Xinxiang Tuoxin Pharm) | 0.04 | 0.04 | 0.04 |
| | Niacinamide (Niacinamide USP from the company Western Drugs) | 2.00 | 2.00 | 2.00 |
| | Hydroxypropyl tetrahydropyrantriol (*) (Mexoryl SCS from the company Novéal) | 4.30 | 4.30 | 4.30 |
| | Phytic acid (Phytic Acid 50% Solution^{®} from the company Tsuno) | 0.15 | 0.08 | 0.15 |
| C | Lauroyl lysine (Amihope^{®} LL from the company Ajinomoto) | 10.00 | 10.0 | - |
| | Zea mays (corn) starch (Maisita 9060 from the company Agrana Starke) | 3.00 | 3.00 | 3.00 |
| D | Ethanol (Ethanol TSDA Cosmos Organic CT from the company Earth Oil) | 5.00 | 4.18 | 5.00 |

| | | | | |
|---|---|---|---|---|
| (*) 70% active material | | | | |

The anti-ageing facial care composition, in the form of a water-in-oil emulsion, 7 according to the invention, and compositions 8 and 9, outside the invention, are prepared in the weight proportions as detailed in Table 4 below. The values are expressed as weight percentages relative to the total weight of the composition.

The protocol for preparing these formulations is identical to that detailed in Example 1 above.

**Table 4**

| Phase | Compound (INCI name) | Formulation 7 according to the invention | Formulation 8 outside the invention | Formulation 9 outside the invention | Formulation 10 outside the invention |
|---|---|---|---|---|---|
| A | Dicaprylyl ether (Cetiol^{®} OE from the company BASF) | 13.00 | 12.00 | 15.00 | 12.00 |
| | C₁₃-C₁₉ Alkane (Emogreen^{®} L15 from the company Evonik) | 3.50 | 4.00 | 4.00 | 4.00 |
| | Undecane (and) Tridecane (Cetiol^{®} UT from the company BASF) | - | 3.00 | - | 3.00 |
| | Polyglyceryl-4 diisostearate/polyhydroxystearate/sebacate (and) caprylic/capric triglyceride (and) glyceryl-3 oleate (and) diisostearyl polyglyceryl-3 dimer dilinoleate (Isolan^{®} 17 MB from the company Evonik) | 3.50 | 4.00 | 4.00 | 4.00 |
| | Polyglyceryl-4 isostearate (Isolan^{®} GI34 from the company Evonik) | 1.70 | 2.00 | 2.00 | 2.00 |
| | Trihydroxystearin (Thixcin^{®} R from the company Elementis) | 0.65 | 1.00 | 0.75 | 0.5 |
| B | Water | qs | qs | qs | qs |
| | Propanediol (1,3-Propanediol from the company Zhangjiagang Glory Chemical) | - | - | - | 3.00 |
| | Glycerin (Glycerine 4811 RSPO MB from the company Oleon) | 5.00 | 5.00 | 5.00 | 5.00 |
| | Magnesium sulfate (Ronacare^{®} Magnesium Sulfate from the company Merck) | 1.74 | 2.00 | 2.00 | 2.00 |
| | Tocopherol (DL α Tocopherol from the company DSM) | 0.005 | - | - | - |
| | Arginine (L-Arginine C Grade from the company Ajinomoto) | - | - | - | 0.20 |
| | Potassium sorbate (Ronacare^{®} Potassium Sorbate from the company Merck) | 0.08 | 0.15 | 0.15 | 0.15 |
| | Pentylene glycol (A-Leen^{®} 5 from the company Minasolve) | 0.80 | 0.80 | 0.80 | - |
| | Salicylic acid (Salicylic Acid from the company JQC) | 0.20 | 0.20 | 0.20 | 0.20 |
| | Phytic acid (Phytic Acid 50% Solution^{®} from the company Tsuno) | 0.15 | 0.15 | 0.15 | 0.15 |
| | Adenosine (Adenosine (L) from the company Xinxiang Tuoxin Pharm) | 0.04 | 0.10 | 0.04 | - |
| | Hydroxypropyl tetrahydropyrantriol (*) (Mexoryl SCS from the company Novéal) | 4.30 | 8.57 | 4.30 | - |
| C | Lauroyl lysine (Amihope^{®} LL from the company Ajinomoto) | 10.00 | 2.00 | - | 2.00 |
| | Zea mays (corn) starch (Maisita 9060 from the company Agrana Starke) | 3.00 | - | 3.00 | - |
| D | Ethanol (Ethanol TSDA Cosmos Organic CT from the company Earth Oil) | 4.35 | 5.00 | 5.00 | 5.00 |
| | Fragrance | - | - | - | 0.21 |

| | | | | | |
|---|---|---|---|---|---|
| (*) 70% active material | | | | | |

### Results

Formulation 1, according to the invention, detailed in Example 1, and formulations 4 to 9 were evaluated as regards their sensory properties and stability after storage for 1 or 2 months at 45°C as detailed in the stability measurement protocol above.

Formulations 1, 4, 5 and 7, in accordance with the invention, show both good stability and good sensory properties.

Formulations 6, 8, 9 and 10, outside the invention, in particular comprising a lauroyl lysine or corn starch filler in a content of less than 6% by weight, do not make it possible to obtain both good sensory properties and acceptable stability. In particular, formulations 6 and 9, although stable, are unsatisfactory in terms of sensory properties.

Formulation 8 is not stable after storage for 2 months at 45°C.

### Example 3

The care compositions, in the form of water-in-oil emulsions, 11 to 15 outside the invention, in particular comprising a filler content of less than 6% by weight, are prepared in the weight proportions as detailed in Table 5 below. The values are expressed as weight percentages relative to the total weight of the composition.

The protocol for preparing these formulations is identical to that detailed in Example 1 above.

**Table 5**

| Phase | Compound (INCI name) | Formulation 11 outside the invention | Formulation 12 outside the invention | Formulation 13 outside the invention | Formulation 14 outside the invention | Formulation 15 outside the invention |
|---|---|---|---|---|---|---|
| A | Dicaprylyl ether (Cetiol^{®} OE from the company BASF) | 12.00 | 12.00 | 12.00 | 15.00 | 12.00 |
| | C₁₃-C₁₉ Alkane (Emogreen^{®} L15 from the company Evonik) | 4.00 | 4.00 | 4.00 | 4.00 | 4.00 |
| | Undecane (and) Tridecane (Cetiol^{®} UT from the company BASF) | 3.00 | 3.00 | - | - | 3.00 |
| | Polyglyceryl-4 diisostearate/polyhy droxystearate/sebac ate (and) caprylic/capric triglyceride (and) glyceryl-3 oleate (and) diisostearyl polyglyceryl-3 dimer dilinoleate (Isolan^{®} 17 MB from the company Evonik) | 4.00 | 4.00 | 4.00 | 4.00 | 4.00 |
| | Polyglyceryl-4 isostearate (Isolan^{®} GI34 from the company Evonik) | 2.00 | 2.00 | 2.00 | 2.00 | 2.00 |
| | Trihydroxystearin (Thixcin^{®} R from the company Elementis) | 0.50 | 0.75 | 1.00 | 0.75 | 1.00 |
| B | Water | qs | qs | qs | qs | qs |
| | Glycerin (Glycerine 4811 RSPO MB from the company Oleon) | 5.00 | 5.00 | 5.00 | 5.00 | - |
| | Magnesium sulfate (Ronacare^{®} Magnesium Sulfate from the company Merck) | 2.00 | 2.00 | 2.00 | 2.00 | 2.00 |
| | Potassium sorbate (Ronacare^{®} Potassium Sorbate from the company Merck) | 0.15 | 0.15 | 0.15 | 0.15 | 0.15 |
| | Pentylene glycol (A-Leen^{®} 5 from the company Minasolve) | 0.80 | 0.80 | 0.80 | 0.80 | 0.80 |
| | Salicylic acid (Salicylic Acid from the company JQC) | 0.20 | 0.20 | 0.20 | 0.20 | 0.20 |
| | Phytic acid (Phytic Acid 50% Solution^{®} from the company Tsuno) | 0.15 | 0.15 | 0.08 | 0.15 | 0.15 |
| | Adenosine (Adenosine (L) from the company Xinxiang Tuoxin Pharm) | | | 0.04 | 0.04 | 0.10 |
| | Ascorbyl glucoside (V2G from the company Macrocare) | - | - | - | - | 2.00 |
| C | Lauroyl lysine (Amihope^{®} LL from the company Ajinomoto) | - | - | - | - | 2.00 |
| | Zea mays (corn) starch (Maisita 9060 from the company Agrana Starke) | - | - | - | 0.80 | - |
| | Talc (Imercare^{®} Pharma from the company Imerys) | 5.00 | 2.00 | - | - | - |
| | Boron nitride (Softouch Boron Nitride Powder CC6058 from the company Momentive Performance Materials) | - | - | 3.00 | - | - |
| D | Ethanol (Ethanol TSDA Cosmos Organic CT from the company Earth Oil) | 5.00 | 5.00 | 5.00 | 5.00 | 5.00 |

### Results

Formulations 11 to 15 do not show satisfactory stability, notably as evaluated after storage for 2 months at 45°C.

### Example 4

The care compositions, in the form of water-in-oil emulsions, 16 to 19 outside the invention, in particular comprising a filler content of less than 6% by weight, are prepared in the weight proportions as detailed in Table 6 below. The values are expressed as weight percentages relative to the total weight of the composition.

The protocol for preparing these formulations is identical to that detailed in Example 1 above.

**Table 6**

| Phase | Compound (INCI name) | Formulation 16 outside the invention | Formulation 17 outside the invention | Formulation 18 outside the invention | Formulation 19 outside the invention |
|---|---|---|---|---|---|
| A | Dicaprylyl ether (Cetiol^{®} OE from the company BASF) | 12.00 | 15.00 | 15.00 | 15.00 |
| | C₁₃-C₁₉ Alkane (Emogreen^{®} L15 from the company Evonik) | 4.00 | 4.00 | 4.00 | 4.00 |
| | Undecane (and) Tridecane (Cetiol^{®} UT from the company BASF) | 3.00 | - | - | - |
| | Polyglyceryl-4 diisostearate/polyhydroxystearate/sebacate (and) caprylic/capric triglyceride (and) glyceryl-3 oleate (and) diisostearyl polyglyceryl-3 dimer dilinoleate (Isolan^{®} 17 MB from the company Evonik) | 4.00 | 4.00 | 4.00 | 4.00 |
| | Polyglyceryl-4 isostearate (Isolan^{®} GI34 from the company Evonik) | 2.00 | 2.00 | 2.00 | 2.00 |
| | Trihydroxystearin (Thixcin^{®} R from the company Elementis) | 1.0 | 0.75 | 0.75 | 0.75 |
| B | Water | qs | qs | qs | qs |
| | Glycerin (Glycerine 4811 RSPO MB from the company Oleon) | 5.00 | 5.00 | 5.00 | 5.00 |
| | Magnesium sulfate (Ronacare^{®} Magnesium Sulfate from the company Merck) | 2.00 | 2.00 | 2.00 | 2.00 |
| | Arginine (L-Arginine C Grade from the company Ajinomoto) | - | 0.18 | 0.18 | - |
| | Potassium sorbate (Ronacare^{®} Potassium Sorbate from the company Merck) | 0.15 | 0.15 | 0.15 | 0.15 |
| | Pentylene glycol (A-Leen^{®} 5 from the company Minasolve) | 0.80 | 0.80 | 0.80 | 0.80 |
| | Salicylic acid (Salicylic Acid from the company JQC) | 0.20 | 0.20 | 0.20 | 0.20 |
| | Phytic acid (Phytic Acid 50% Solution^{®} from the company Tsuno) | 0.15 | 0.15 | 0.15 | 0.15 |
| | Adenosine (Adenosine (L) from the company Xinxiang Tuoxin Pharm) | - | 0.04 | 0.04 | 0.04 |
| | Niacinamide (Niacinamide USP PC from the company Lonza) | - | - | 2.00 | - |
| | Lactic acid (L-Lactic Acid (90%) (CRG) from the company Musashino Chemical Laboratory) | - | - | - | 6.00 |
| | Ascorbyl glucoside (V2G from the company Macrocare) | - | 1.0 | - | - |
| | Sodium citrate (Trisodium Citrate Dihydrate Fine Granular from the company Citrique Belge) | - | 0.35 | - | - |
| | Sodium hydroxide (Lessive Soude 30% CPP from the company Quaron) | - | - | - | 0.50 |
| C | Zea mays (corn) starch (Maisita 9060 from the company Agrana Starke) | 3.00 | 3.00 | 3.00 | 3.00 |
| D | Ethanol (Ethanol TSDA Cosmos Organic CT from the company Earth Oil) | 5.00 | 5.00 | 5.00 | 5.00 |

### Results

Formulations 16 to 19 do not have satisfactory sensory properties.

Formulations 17 and 18 have satisfactory stability but formulation 18 is not stable since it undergoes coalescence after storage for 2 months at 45°C.

### Example 5

The care compositions, in the form of water-in-oil emulsions, 20 and 21 outside the invention, in particular comprising fillers different from those required according to the invention in a content of less than 6% by weight, are prepared, in the weight proportions as detailed in Table 7 below. The values are expressed as weight percentages relative to the total weight of the composition.

The protocol for preparing these formulations is identical to that detailed in Example 1 above.

**Table 7**

| Phase | Compound (INCI name) | Formulation 20 outside the invention | Formulation 21 outside the invention |
|---|---|---|---|
| A | Dicaprylyl ether (Cetiol^{®} OE from the company BASF) | 12.00 | 12.00 |
| | C₁₅-C₁₉ Alkane (Emogreen^{®} L15 from the company Evonik) | 4.00 | 4.00 |
| | Undecane (and) Tridecane (Cetiol^{®} UT from the company BASF) | 3.00 | 3.00 |
| | Polyglyceryl-4 diisostearate/polyhydroxystearate/sebacate (and) caprylic/capric triglyceride (and) glyceryl-3 oleate (and) diisostearyl polyglyceryl-3 dimer dilinoleate (Isolan^{®} 17 MB from the company Evonik) | 4.00 | 4.00 |
| | Polyglyceryl-4 isostearate (Isolan^{®} GI34 from the company Evonik) | 2.00 | 2.00 |
| | Trihydroxystearin (Thixcin^{®} R from the company Elementis) | 1.0 | 0.50 |
| B | Water | qs | qs |
| | Glycerin (Glycerine 4811 RSPO MB from the company Oleon) | 5.00 | 5.00 |
| | Magnesium sulfate (Ronacare^{®} Magnesium Sulfate from the company Merck) | 2.00 | 2.00 |
| | Potassium sorbate (Ronacare^{®} Potassium Sorbate from the company Merck) | 0.15 | 0.15 |
| | Pentylene glycol (A-Leen^{®} 5 from the company Minasolve) | 0.80 | 0.80 |
| | Salicylic acid (Salicylic Acid from the company JQC) | 0.20 | 0.20 |
| | Phytic acid (Phytic Acid 50% Solution^{®} from the company Tsuno) | 0.15 | 0.15 |
| C | Lauroyl lysine (Amihope^{®} LL from the company Ajinomoto) | - | 2.00 |
| | Cellulose (Cellulobeads USF from the company Daito Kasei Kogyo) | 2.00 | - |
| | Perlite (Optimat 2550 OR from the company Imerys) | - | 0.10 |
| D | Ethanol (Ethanol TSDA Cosmos Organic CT from the company Earth Oil) | 5.00 | 5.00 |

### Results

Formulations 20 and 21 do not show satisfactory stability.

## Claims

1. Composition, notably a cosmetic composition, in particular for making up and/or caring for keratin materials, comprising:
- at least one continuous fatty phase;
- at least one aqueous phase at a concentration of at least 50.0% by weight, relative to the total weight of the composition, and dispersed in said fatty phase;
- at least two esters that are different from each other, chosen from esters of polyglycerol with at least one fatty acid containing from 8 to 30 carbon atoms;
- glyceryl trihydroxystearate; and
- at least 6.0% by weight, relative to the total weight of the composition, of at least one filler chosen from talc, hydrophobic silica aerogel particles, C₈-C₂₂ N-acylamino acid particles, modified or unmodified starches, boron nitride, polymeric fillers, metal oxides and mixtures thereof.

2. Composition according to Claim 1, **characterized in that** the esters of polyglycerol with at least one fatty acid containing from 8 to 30 carbon atoms are chosen from:
- at least one ester of polyglycerol with at least one poly(hydroxystearic acid) and carboxylic acids, resulting from the esterification between at least one polyglycerol and (i) at least one poly(hydroxystearic acid); (ii) at least one di- and/or tricarboxylic acid; and (iii) at least one saturated or unsaturated, linear or branched fatty acid containing from 6 to 22 carbon atoms; and
- at least one ester of polyglycerol including from 4 to 15 glycerol units and of at least one saturated or unsaturated, linear or branched fatty acid including from 6 to 24 carbon atoms.

3. Composition according to Claim 2, **characterized in that** the ester of polyglycerol with a poly(hydroxystearic acid) and carboxylic acids is derived from esterification between a mixture of polyglycerol and (i) a poly(hydroxystearic acid) with 4 polyglycerol units; (ii) linear or branched, aliphatic dicarboxylic acids containing 8 to 12 carbon atoms; and (iii) saturated or unsaturated, linear or branched fatty acids containing from 16 to 20 carbon atoms, and preferably is polyglyceryl-4 diisostearate/polyhydroxystearate/sebacate.

4. Composition according to Claim 2, **characterized in that** the ester of polyglycerol and of fatty acid is chosen from esters derived from the esterification reaction between a polyglycerol comprising from 4 to 5 glycerol units, in particular comprising 4 glycerol units, and at least one aliphatic monocarboxylic acid comprising an alkyl chain containing from 16 to 20 carbon atoms, such as a stearyl and/or isostearyl chain.

5. Composition according to any one of the preceding claims, comprising between 0.5% and 15% by weight, preferably between 1.5% and 10% by weight, more preferentially between 2.5% and 8.0% by weight of esters of polyglycerol with at least one fatty acid containing from 8 to 30 carbon atoms, relative to the total weight of the composition.

6. Composition according to any one of the preceding claims, comprising between 0.01% and 2.0% by weight, in particular between 0.1% and 1.5% by weight, preferably between 0.1% and 1.0% by weight, more preferentially between 0.2% and 0.8% by weight, of glyceryl trihydroxystearate, relative to the total weight of the composition.

7. Composition according to any one of the preceding claims, in which the filler is chosen from talc, hydrophobic silica aerogel particles, C₈-C₂₂ N-acylamino acid particles, modified or unmodified starches, boron nitride, and mixtures thereof, more preferentially chosen from C₈-C₂₂ N-acylamino acid particles, modified or unmodified starches, and mixtures thereof.

8. Composition according to any one of the preceding claims, in which the filler(s) are present in a content ranging from 6.0% to 60% by weight, in particular from 8.0% to 50% and more particularly from 10% to 30% by weight relative to the total weight of the composition.

9. Composition according to any one of the preceding claims, comprising between 5.0% and 50% by weight of fatty phase, in particular between 10% and 30% by weight, and preferably between 15% and 25% by weight, relative to the total weight of the composition.

10. Composition according to any one of the preceding claims, comprising less than 2.0% by weight of silicone oil(s), in particular less than 1.0% by weight, preferably less than 0.5% by weight, relative to the total weight of the composition, and more preferentially being free of silicone oil(s).

11. Composition according to any one of the preceding claims, comprising at least one oil chosen from mixtures of linear and/or branched C₁₅-C₁₉ alkanes, synthetic ethers containing from 10 to 40 carbon atoms, in particular dicaprylyl ether, and mixtures thereof.

12. Composition according to any one of the preceding claims, comprising from 50% to 95% by weight of aqueous phase, preferably from 50% to 85% by weight, and more preferentially from 50% to 75% by weight, relative to the total weight of said composition.

13. Composition according to any one of the preceding claims, also comprising at least one cosmetic active agent, in particular at least one anti-ageing cosmetic active agent notably chosen from hydroxypropyltetrahydropyrantriol, adenosine, niacinamide compounds, salicylic acid compounds, ascorbic acid, and mixtures thereof, more particularly chosen from hydroxypropyltetrahydropyrantriol, adenosine, niacinamide compounds, and mixtures thereof.

14. Cosmetic process for caring for keratin materials, in particular the skin, comprising at least one step of applying to said keratin materials a composition as defined according to any one of Claims 1 to 13.

15. Use of a composition as defined according to any one of Claims 1 to 13, for combating the signs of ageing of the skin.

## Patentansprüche

1. Zusammensetzung, insbesondere kosmetische Zusammensetzung, insbesondere zum Schminken und/oder Pflegen von Keratinmaterialien, umfassend:
- mindestens eine kontinuierliche Fettphase;
- mindestens eine wässrige Phase in einer Konzentration von mindestens 50,0 Gew.-%, bezogen auf das Gesamtgewicht der Zusammensetzung, die in der Fettphase dispergiert ist;
- mindestens zwei voneinander verschiedene Ester, die aus Estern von Polyglycerin mit mindestens einer Fettsäure mit 8 bis 30 Kohlenstoffatomen ausgewählt sind;
- Glyceryltrihydroxystearat; und
- mindestens 6,0 Gew.-%, bezogen auf das Gesamtgewicht der Zusammensetzung, mindestens eines Füllstoffs, der aus Talk, hydrophoben Siliciumdioxid-Aerogelteilchen, C₈-C₂₂-N-Acylaminosäure-Teilchen, modifizierten oder unmodifizierten Stärken, Bornitrid, polymeren Füllstoffen, Metalloxiden und Mischungen davon ausgewählt ist.

2. Zusammensetzung nach Anspruch 1, **dadurch gekennzeichnet, dass** die Ester von Polyglycerin mit mindestens einer Fettsäure mit 8 bis 30 Kohlenstoffatomen ausgewählt sind aus:
- mindestens einem Ester von Polyglycerin mit mindestens einer Poly(hydroxystearinsäure) und Carbonsäuren, der aus der Veresterung zwischen mindestens einem Polyglycerin und (i) mindestens einer Poly(hydroxystearinsäure); (ii) mindestens einer Di- und/oder Tricarbonsäure und (iii) mindestens einer gesättigten oder ungesättigten, linearen oder verzweigten Fettsäure mit 6 bis 22 Kohlenstoffatomen resultiert; und
- mindestens einem Ester von Polyglycerin mit 4 bis 15 Glycerin-Einheiten und mindestens einer gesättigten oder ungesättigten, linearen oder verzweigten Fettsäure mit 6 bis 24 Kohlenstoffatomen.

3. Zusammensetzung nach Anspruch 2, **dadurch gekennzeichnet, dass** sich der Ester von Polyglycerin mit einer Poly(hydroxystearinsäure) und Carbonsäuren von der Veresterung zwischen einer Mischung von Polyglycerin und (i) einer Poly(hydroxystearinsäure) mit 4 Polyglycerin-Einheiten; (ii) linearen oder verzweigten, aliphatischen Dicarbonsäuren mit 8 bis 12 Kohlenstoffatomen und (iii) gesättigten oder ungesättigten, linearen oder verzweigten Fettsäuren mit 16 bis 20 Kohlenstoffatomen ableitet und vorzugsweise Polyglyceryl-4 Diisostearate/Polyhydroxystearate/Sebacate ist.

4. Zusammensetzung nach Anspruch 2, **dadurch gekennzeichnet, dass** der Ester von Polyglycerin und Fettsäure aus Estern, die sich von der Veresterungsreaktion zwischen einem Polyglycerin mit 4 bis 5 Glycerin-Einheiten, insbesondere mit 4 Glycerin-Einheiten, und mindestens einer aliphatischen Monocarbonsäure mit einer Alkylkette mit 16 bis 20 Kohlenstoffatomen, wie einer Stearyl- und/oder Isostearylkette, ableiten, ausgewählt ist.

5. Zusammensetzung nach einem der vorhergehenden Ansprüche, umfassend zwischen 0,5 und 15 Gew.-%, vorzugsweise zwischen 1,5 und 10 Gew.-%, weiter bevorzugt zwischen 2,5 und 8,0 Gew.-%, Ester von Polyglycerin mit mindestens einer Fettsäure mit 8 bis 30 Kohlenstoffatomen, bezogen auf das Gesamtgewicht der Zusammensetzung.

6. Zusammensetzung nach einem der vorhergehenden Ansprüche, umfassend zwischen 0,01 und 2,0 Gew.-%, insbesondere zwischen 0,1 und 1,5 Gew.-%, vorzugsweise zwischen 0,1 und 1,0 Gew.-%, weiter bevorzugt zwischen 0,2 und 0,8 Gew.-%, Glyceryltrihydroxystearat, bezogen auf das Gesamtgewicht der Zusammensetzung.

7. Zusammensetzung nach einem der vorhergehenden Ansprüche, wobei der Füllstoff aus Talk, hydrophoben Siliciumdioxid-Aerogelteilchen, C₈-C₂₂-N-Acylaminosäure-Teilchen, modifizierten oder unmodifizierten Stärken, Bornitrid und Mischungen davon ausgewählt ist und weiter bevorzugt aus C₈-C₂₂-N-Acylaminosäure-Teilchen, modifizierten oder unmodifizierten Stärken und Mischungen davon ausgewählt ist.

8. Zusammensetzung nach einem der vorhergehenden Ansprüche, wobei der Füllstoff bzw. die Füllstoffe in einem Gehalt im Bereich von 6,0 bis 60 Gew.-%, insbesondere von 8,0 bis 50 Gew.-% und spezieller von 10 bis 30 Gew.-%, bezogen auf das Gesamtgewicht der Zusammensetzung, vorliegt bzw. vorliegen.

9. Zusammensetzung nach einem der vorhergehenden Ansprüche, umfassend zwischen 5,0 und 50 Gew.-% Fettphase, insbesondere zwischen 10 und 30 Gew.-% und vorzugsweise zwischen 15 und 25 Gew.-%, bezogen auf das Gesamtgewicht der Zusammensetzung.

10. Zusammensetzung nach einem der vorhergehenden Ansprüche, umfassend weniger als 2,0 Gew.-% Silikonöl(e), insbesondere weniger als 1,0 Gew.-%, vorzugsweise weniger als 0,5 Gew.-%, bezogen auf das Gesamtgewicht der Zusammensetzung, wobei die Zusammensetzung weiter bevorzugt frei von Silikonöl(en) ist.

11. Zusammensetzung nach einem der vorhergehenden Ansprüche, umfassend mindestens ein Öl, das aus Mischungen von linearen und/oder verzweigten C₁₅-C₁₉-Alkanen, synthetischen Ethern mit 10 bis 40 Kohlenstoffatomen, insbesondere Dicaprylylether, und Mischungen davon ausgewählt ist.

12. Zusammensetzung nach einem der vorhergehenden Ansprüche, umfassend 50 bis 95 Gew.-% wässrige Phase, vorzugsweise 50 bis 85 Gew.-% und weiter bevorzugt 50 bis 75 Gew.-%, bezogen auf das Gesamtgewicht der Zusammensetzung.

13. Zusammensetzung nach einem der vorhergehenden Ansprüche, ebenfalls umfassend mindestens einen kosmetischen Wirkstoff, insbesondere mindestens einen kosmetischen Anti-Aging-Wirkstoff, insbesondere ausgewählt aus Hydroxypropyltetrahydropyrantriol, Adenosin, Niacinamidverbindungen, Salicylsäureverbindungen, Ascorbinsäure und Mischungen davon, spezieller ausgewählt aus Hydroxypropyltetrahydropyrantriol, Adenosin, Niacinamidverbindungen und Mischungen davon.

14. Kosmetisches Verfahren zum Pflegen von Keratinmaterialien, insbesondere der Haut, umfassend mindestens einen Schritt des Aufbringens einer Zusammensetzung gemäß einem der Ansprüche 1 bis 13 auf die Keratinmaterialien.

15. Verwendung einer Zusammensetzung gemäß einem der Ansprüche 1 bis 13 zur Bekämpfung der Anzeichen von Hautalterung.

## Revendications

1. Composition, notamment composition cosmétique, en particulier pour le maquillage et/ou le soin des matières kératiniques, comprenant :
- au moins une phase grasse continue ;
- au moins une phase aqueuse à une concentration d'au moins 50,0 % en poids par rapport au poids total de la composition, et dispersée dans ladite phase grasse ;
- au moins deux esters différents l'un de l'autre, choisis parmi les esters de polyglycérol avec au moins un acide gras contenant de 8 à 30 atomes de carbone ;
- du trihydroxystéarate de glycéryle ; et
- au moins 6,0 % en poids, par rapport au poids total de la composition, d'au moins une charge choisie parmi le talc, les particules d'aérogel de silice hydrophobes, les particules de N-acylaminoacides en C₈-C₂₂, les amidons modifiés ou non modifiés, le nitrure de bore, les charges polymériques, les oxydes métalliques et leurs mélanges.

2. Composition selon la revendication 1, **caractérisée en ce que** les esters de polyglycérol avec au moins un acide gras contenant de 8 à 30 atomes de carbone sont choisis parmi :
- au moins un ester de polyglycérol avec au moins un poly(acide hydroxystéarique) et des acides carboxyliques, résultant de l'estérification entre au moins un polyglycérol et (i) au moins un poly(acide hydroxystéarique) ; (ii) au moins un acide di- et/ou tricarboxylique ; et (iii) au moins un acide gras saturé ou insaturé, linéaire ou ramifié, contenant de 6 à 22 atomes de carbone ; et
- au moins un ester de polyglycérol comportant de 4 à 15 motifs glycérol et d'au moins un acide gras saturé ou insaturé, linéaire ou ramifié comportant de 6 à 24 atomes de carbone.

3. Composition selon la revendication 2, **caractérisée en ce que** l'ester de polyglycérol avec un poly(acide hydroxystéarique) et des acides carboxyliques est dérivé de l'estérification entre un mélange de polyglycérol et (i) un poly(acide hydroxystéarique) ayant 4 motifs polyglycérol ; (ii) des acides dicarboxyliques aliphatiques linéaires ou ramifiés contenant 8 à 12 atomes de carbone ; et (iii) des acides gras saturés ou insaturés, linéaires ou ramifiés contenant de 16 à 20 atomes de carbone, et de préférence est le polyglycéryl-4 diisostéarate/polyhydroxystéarate/sébacate.

4. Composition selon la revendication 2, **caractérisée en ce que** l'ester de polyglycérol et d'acide gras est choisi parmi les esters dérivés de la réaction d'estérification entre un polyglycérol comprenant de 4 à 5 motifs glycérol, en particulier comprenant 4 motifs glycérol, et au moins un acide monocarboxylique aliphatique comprenant une chaîne alkyle contenant de 16 à 20 atomes de carbone, telle qu'une chaîne stéaryle et/ou isostéaryle.

5. Composition selon l'une quelconque des revendications précédentes, comprenant entre 0,5 % et 15 % en poids, de préférence entre 1,5 % et 10 % en poids, plus préférentiellement entre 2,5 % et 8,0 % en poids d'esters de polyglycérol avec au moins un acide gras contenant de 8 à 30 atomes de carbone, par rapport au poids total de la composition.

6. Composition selon l'une quelconque des revendications précédentes, comprenant entre 0,01 % et 2,0 % en poids, en particulier entre 0,1 % et 1,5 % en poids, de préférence entre 0,1 % et 1,0 % en poids, plus préférentiellement entre 0,2 % et 0,8 % en poids, de trihydroxystéarate de glycéryle, par rapport au poids total de la composition.

7. Composition selon l'une quelconque des revendications précédentes, dans laquelle la charge est choisie parmi le talc, les particules d'aérogel de silice hydrophobes, les particules de N-acylaminoacides en C₈-C₂₂, les amidons modifiés ou non modifiés, le nitrure de bore, et leurs mélanges, plus préférentiellement choisis parmi les particules de N-acylaminoacides en C₈-C₂₂, les amidons modifiés ou non modifiés, et leurs mélanges.

8. Composition selon l'une quelconque des revendications précédentes, dans laquelle la ou les charges sont présentes en une teneur allant de 6,0 % à 60 % en poids, en particulier de 8,0 % à 50 % et plus particulièrement de 10 % à 30 % en poids par rapport au poids total de la composition.

9. Composition selon l'une quelconque des revendications précédentes, comprenant entre 5,0 % et 50 % en poids de phase grasse, en particulier entre 10 % et 30 % en poids, et de préférence entre 15 % et 25 % en poids, par rapport au poids total de la composition.

10. Composition selon l'une quelconque des revendications précédentes, comprenant moins de 2,0 % en poids d'huile(s) de silicone, en particulier moins de 1,0 % en poids, de préférence moins de 0,5 % en poids, par rapport au poids total de la composition, et plus préférentiellement exempte d'huile(s) de silicone.

11. Composition selon l'une quelconque des revendications précédentes, comprenant au moins une huile choisie parmi les mélanges d'alcanes linéaires et/ou ramifiés en C₁₅-C₁₉, les éthers synthétiques contenant de 10 à 40 atomes de carbone, en particulier l'éther dicaprylylique, et leurs mélanges.

12. Composition selon l'une quelconque des revendications précédentes, comprenant de 50 % à 95 % en poids de phase aqueuse, de préférence de 50 % à 85 % en poids, et plus préférentiellement de 50 % à 75 % en poids, par rapport au poids total de ladite composition.

13. Composition selon l'une quelconque des revendications précédentes, comprenant en outre au moins un agent actif cosmétique, en particulier au moins un agent actif cosmétique anti-âge notamment choisi parmi l'hydroxypropyltétrahydropyranetriol, l'adénosine, les composés de niacinamide, les composés de l'acide salicylique, l'acide ascorbique, et leurs mélanges, plus particulièrement choisi parmi l'hydroxypropyltétrahydropyranetriol, l'adénosine, les composés de niacinamide, et leurs mélanges.

14. Procédé cosmétique pour le soin des matières kératiniques, en particulier la peau, comprenant au moins une étape d'application sur lesdites matières kératiniques d'une composition telle que définie selon l'une quelconque des revendications 1 à 13.

15. Utilisation d'une composition telle que définie selon l'une quelconque des revendications 1 à 13, pour lutter contre les signes de vieillissement de la peau.
